(19) Europäisches Patentamt / European Patent Office / Office européen des brevets

(11) **EP 3 606 483 B1**

(12) **EUROPÄISCHE PATENTSCHRIFT**

(45) Veröffentlichungstag und Bekanntmachung des Hinweises auf die Patenterteilung:
**22.02.2023   Patentblatt 2023/08**

(21) Anmeldenummer: **18717861.1**

(22) Anmeldetag: **29.03.2018**

(51) Internationale Patentklassifikation (IPC):
**A61F 9/008** *(2006.01)*       **A61F 9/009** *(2006.01)*

(52) Gemeinsame Patentklassifikation (CPC):
**A61F 9/009; A61F 9/00825;** A61F 2009/0087;
A61F 2009/00872; A61F 2009/00887;
A61F 2009/00889

(86) Internationale Anmeldenummer:
**PCT/EP2018/058189**

(87) Internationale Veröffentlichungsnummer:
**WO 2018/185009 (11.10.2018 Gazette 2018/41)**

(54) **KURZPULS-LASER-AUGENCHIRURGIESYSTEM ZUM BEHANDELN EINES AUGES UND VERFAHREN ZUM BESTIMMEN EINES FÜLLSTANDS EINER FLÜSSIGKEIT IN EINEM HOHLRAUM EINES KURZPULS-LASER-AUGENCHIRURGIESYSTEMS**

SHORT-PULSE LASER EYE-SURGERY SYSTEM FOR TREATING AN EYE AND METHOD FOR DETERMINING THE FILL LEVEL OF A LIQUID IN A HOLLOW CHAMBER OF A SHORT-PULSE LASER EYE-SURGERY SYSTEM

SYSTÈME DE CHIRURGIE OCULAIRE LASER À IMPULSIONS COURTES PERMETTANT DE TRAITER UN OEIL ET PROCÉDÉ DE DÉTERMINATION DE NIVEAU DE REMPLISSAGE D'UN LIQUIDE DANS UNE CAVITÉ D'UN SYSTÈME DE CHIRURGIE OCULAIRE LASER À IMPULSIONS COURTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.04.2017   DE 102017107260**

(43) Veröffentlichungstag der Anmeldung:
**12.02.2020   Patentblatt 2020/07**

(73) Patentinhaber: **Carl Zeiss Meditec AG**
**07745 Jena (DE)**

(72) Erfinder:
• **RILL, Michael Stefan**
**07751 Jena (DE)**
• **LAQUA, Stephan**
**07745 Jena (DE)**
• **NIKOLAEV, Alexander**
**07747 Jena (DE)**

(74) Vertreter: **Wenzel Nemetzade Warthmüller**
**Patentanwälte Part mbB**
**Maximilianstraße 2**
**80539 München (DE)**

(56) Entgegenhaltungen:
WO-A1-2016/100439       WO-A2-2016/058931
US-A1- 2013 103 014       US-A1- 2016 175 146
US-A1- 2017 056 243

EP 3 606 483 B1

**Beschreibung**

Die Erfindung betrifft ein Kurzpuls-Laser-Augenchirurgiesystem zum Behandeln eines Auges und ein Verfahren zum Bestimmen eines Füllstands einer Flüssigkeit in einem Hohlraum eines Kurzpuls-Laser-Augenchirurgiesystems.

Stand der Technik

Bei der Behandlung des Auges mit einem Kurzpuls-Laser, insbesondere bei der lasergestützten Kataraktchirurgie, muss die geometrische bzw. räumliche Lage des Auges des zu behandelnden Patienten relativ zu dem Fokus des Laserstrahls genau definiert sein bzw. bekannt sein. Da bei der Behandlung bzw. Operation des Auges lediglich eine lokale Anästhesie bzw. eine Tropfanästhesie verwendet wird, kann es vorkommen, dass der Patient sein Auge bewusst oder unbewusst (sogenannten Mikrosakkaden) (relativ zu einem Kurzpuls-Laser-Augenchirurgiegerät bzw. relativ zu einem Kurzpuls-Laser-Augenchirurgiesystem) bewegt. Daher wird das Auge des Patienten mittels eines Patienteninterfaces an das Kurzpuls-Laser-Augenchirurgiegerät mechanisch gekoppelt bzw. an diesem fixiert. Das Patienteninterface wird zwischen dem Kurzpuls-Laser-Augenchirurgiegerät und dem Auge des Patienten angeordnet.

Oft wird ein sogenanntes Flüssigkeits-Patienteninterface als Patienteninterface verwendet. Hierbei ist ein Hohlraum mit einer physiologischen Salzlösung (BSS; balanced salt solution) gefüllt, die mit dem Auge in Kontakt steht. Der Hohlraum ist auf der dem Kurzpuls-Laser-Augenchirurgiegerät abgewandten Seite offen und mit dem Auge verschließbar. Die Salzlösung hält das Patientenauge feucht und nivelliert den Brechungsindex zwischen einem Ausgangselement bzw. einer Ausgangslinse des Patienteninterfaces, die den Hohlraum in Richtung des Kurzpuls-Laser-Augenchirurgiegeräts begrenzt, und dem Auge, so dass wenig bis keine Reflexion und/oder Rückstreuung und/oder Streuung auftritt, wenn der Hohlraum mit der Flüssigkeit vollständig gefüllt ist und der Therapiestrahl aus dem Kurzpuls-Laser-Augenchirurgiegerät durch das Ausgangselement hindurch in das Auge propagiert.

Vor dem Beginn der Operation bzw. Behandlung des Auges mit dem Laser des Kurzpuls-Laser-Augenchirurgiesystems wird (nach dem Verschließen des Hohlraums mit dem Auge) der Hohlraum vollständig mit der Flüssigkeit (z.B. der physiologischen Salzlösung BSS) gefüllt. Es besteht jedoch die Gefahr, dass vor oder während der Operation bzw. Behandlung des Auges die Salzlösung teilweise oder vollständig aus dem Hohlraum ausfließt bzw. austritt. Dies kann zu undefinierten optischen Artefakten bzw. Effekten führen, die die Behandlung des Auges mit dem Laser unter Umständen negativ beeinträchtigen. Ein Ausfließen kann u.a. durch Undichtigkeit der Dichtungen des Hohlraums und/oder durch kurzzeitiges partielles Entfernen des Auges von dem Hohlraum entstehen.

Daher ist das Überwachen bzw. Bestimmen des Füllstands bzw. Spiegels der Flüssigkeit bzw. der Salzlösung in dem Hohlraum für eine sichere Behandlung des Auges mit dem Laser des Kurzpuls-Laser-Augenchirurgiesystems notwendig.

Ein System bzw. Verfahren, das den Füllstand einer Flüssigkeit in einem Patienteninterface mittels OCT misst, ist aus Dokument US 2013/103014 bekannt.

Bisher wird der Füllstand bzw. Spiegel der Flüssigkeit in dem Hohlraum mittels elektrischer und/oder elektro-akustischer Verfahren bestimmt bzw. überwacht. Hierbei wird z.B. ein durch die Flüssigkeit in dem Hohlraum hergestellter elektrischer Kontakt zwischen zwei leitenden Elementen des Patienteninterfaces überwacht. Nachteilig an diesen Verfahren ist, dass die Herstellung des Patienteninterface in der Regel aufwendig und teuer ist, da diese Patienteninterfaces elektrische und/oder elektro-akustische Elemente aufweisen. Dies führt typischerweise zu hohen Kosten, da das Patienteninterface bei jedem Patienten ausgetauscht werden muss, um Sterilität zu gewährleisten. Zudem stehen die elektrischen und/oder elektro-akustischen Komponenten des Patienteninterfaces in indirektem Kontakt mit dem Auge, was besondere Anforderungen für die notwendige Sterilisation nach sich zieht. Übliche Sterilisationsverfahren mittels reaktiver Gase und/oder Flüssigkeiten sind in der Regel nicht durchführbar, da diese die elektrischen und/oder elektro-akustischen Komponenten bzw. deren metallischen Elemente angreifen und/oder korrodieren, wodurch in der Regel die Messung zur Bestimmung des Füllstands bzw. Spiegels der Flüssigkeit negativ beeinträchtigt wird. Darüber hinaus muss bei bisherigen Verfahren, bei denen elektrisch und/oder elektro-akustisch der Füllstand bzw. Spiegel der Flüssigkeit im Hohlraum bestimmt wird, in der Regel mindestens ein elektrischer Anschluss an dem Patienteninterface vorhanden sein. Dies erhöht einerseits die Komplexität des Patienteninterfaces weiter und führt andererseits unter ungünstigen Umständen zu zusätzlichen Sicherheitsrisiken, z.B. in Form von Ableitströmen durch das Patientenauge. Zudem muss das Patienteninterface über den elektrischen Anschluss durch den Arzt oder durch anderes Personal vor der Durchführung der Operation/Behandlung mit weiteren Elementen elektrisch verbunden werden. Dies erhöht in der Regel den Aufwand.

Daher liegt der Erfindung die Aufgabe zu Grunde, ein Kurzpuls-Laser-Augenchirurgiesystem zum Behandeln eines Auges aufzuzeigen, welches zum Bestimmen des Füllstands bzw. Spiegels der Flüssigkeit in einem Hohlraum eines Flüssigkeits-Patienteninterfaces ausgebildet ist und technisch einfach aufgebaut sowie kostengünstig herstellbar ist. Des Weiteren liegt der Erfindung die Aufgabe zu Grunde, ein Verfahren zum Bestimmen eines Füllstands bzw. Spiegels einer Flüssigkeit in einem Hohlraum eines Kurzpuls-Laser-Augenchirurgiesystems aufzuzeigen, welches technisch einfach ist und mit technisch einfachen und kostengünstigen Flüssigkeits-Patienteninterfaces durchführbar ist.

Diese Aufgaben werden jeweils durch ein Kurzpuls-Laser-Augenchirurgiesystem gemäß Anspruch 1 bzw. ein Verfahren gemäß Anspruch 9 gelöst.

Insbesondere wird die Aufgabe durch ein Kurzpuls-Laser-Augenchirurgiesystem zum Behandeln eines Auges gelöst. Das Kurzpuls-Laser-Augenchirurgiesystem umfasst ein Kurzpuls-Laser-Augenchirurgiegerät zum Erzeugen eines Laserstrahls zur Behandlung des Auges und ein Flüssigkeits-Patienteninterface zum Fixieren des Auges. Das Flüssigkeits-Patienteninterface kann zum Kontaktieren eines Auges dienen, das mittels des Laserstrahls des Kurzpuls-Laser-Augenchirurgiegeräts behandelt werden kann. Das Flüssigkeits-Patienteninterface kann das Auge in Bezug auf das Kurzpuls-Laser-Augenchirurgiegerät arretieren.

Das Flüssigkeits-Patienteninterface umfasst einen Hohlraum zum Aufnehmen einer Flüssigkeit. Der Hohlraum ist entlang einer Richtung des Laserstrahls nach einem Ausgangselement des Flüssigkeits-Patienteninterfaces angeordnet und ist auf einer von dem Ausgangselement abgewandten Seite offen und von dem Auge verschließbar. Das Kurzpuls-Laser-Augenchirurgiegerät weist eine Strahlerzeugungsvorrichtung zum Aussenden von optischen Messstrahlen aus dem Kurzpuls-Laser-Augenchirurgiegerät in das Flüssigkeits-Patienteninterface auf. Die optischen Messstrahlen können sichtbares Licht und/oder nicht-sichtbares Licht, insbesondere Infrarotlicht, sein. Die optischen Messstrahlen können kohärentes und/oder nicht-kohärentes Licht umfassen. Die Strahlerzeugungsvorrichtung kann eine Lasererzeugungsvorrichtung zum Erzeugen eines Laserstrahls sein. Der Laserstrahl der Strahlerzeugungsvorrichtung kann der Laserstrahl sein, der für die Behandlung des Auges verwendet wird. Denkbar ist ebenfalls, dass der Laserstrahl der Strahlerzeugungsvorrichtung sich von dem Laserstrahl zum Behandeln des Auges unterscheidet. Auch vorstellbar ist, dass die Strahlerzeugungsvorrichtung sichtbares nicht-kohärentes Licht abstrahlt, z.B. mittels einer Glühlampe und/oder einer Gasentladungslampe und/oder einer LED. Das Ausgangselement des Flüssigkeits-Patienteninterfaces ist für die von dem Kurzpuls-Laser-Augenchirurgiegerät ausgesandten optischen Messstrahlen, die Reflexion und/oder die Rückstreuung und den Laserstrahl zumindest teilweise, insbesondere vollständig, durchlässig ausgebildet.

Das Kurzpuls-Laser-Augenchirurgiegerät weist zudem eine Erfassungsvorrichtung zum Erfassen von Reflexion und/oder Rückstreuung und/oder Transmission der optischen Messstrahlen aus dem Flüssigkeits-Patienteninterface in dem Kurzpuls-Laser-Augenchirurgiegerät auf. Die Erfassungsvorrichtung kann ein optischer Sensor und/oder eine Kamera und/oder ein charge-coupled-device (CCD) und/oder ein Helligkeitssensor umfassen oder sein. Die Erfassungsvorrichtung kann zum Erfassen bzw. Detektieren von (reflektiertem und/oder rückgestreutem und/oder transmittiertem) sichtbarem und/oder nicht-sichtbarem Licht, z.B. Infrarotlicht, ausgelegt sein. Die Reflexion und/oder Rückstreuung kann alles Licht oder einen Teil des Lichts umfassen, das von dem Flüssigkeits-Patienteninterface und/oder dem Auge in das Kurzpuls-Laser-Augenchirurgiegerät zurückgeworfen wird. Transmittiertes Licht kann das Licht sein, das entlang einer Erstreckungsrichtung des Ausgangselements durch das Ausgangselement gelangt ist.

Das Kurzpuls-Laser-Augenchirurgiegerät umfasst darüber hinaus eine Bestimmungsvorrichtung zum Bestimmen mindestens eines ersten Messwerts aus der erfassten Reflexion und/oder Rückstreuung und/oder Transmission der optischen Messstrahlen. Die Bestimmungsvorrichtung kann den ersten Messwert beispielsweise ermitteln oder berechnen. Hierfür wird die erfasste Reflexion und/oder Rückstreuung und/oder Transmission verarbeitet. Die Bestimmungsvorrichtung kann z.B. einen Prozessor/IC und/oder einen Computer und/oder eine analoge und/oder digitale Schaltung umfassen oder sein. Der mindestens eine erste Messwert kann ein direkt aus der Erfassungsvorrichtung stammender Wert, wie z.B. ein gemessener Strom eines Helligkeitssensors, sein. Alternativ kann der mindestens erste Messwert ein aus dem von der Erfassungsvorrichtung erfassten Wert bzw. Werten abgeleiteter bzw. berechneter Messwert sein. Beispielsweise kann der mindestens eine erste Messwert eine optische Weglänge zwischen zwei Elementen des Flüssigkeits-Patienteninterfaces und/oder ein durchschnittlicher Helligkeitswert bzw. Intensitätswert eines von der Erfassungsvorrichtung aufgenommenen Bilds sein. Der mindestens eine erste Messwert kann auch mehrere erste Messwerte umfassen. Der mindestens eine erste Messwert kann z.B. ein 2D- oder 3D-Digitalbild sein, das von der Erfassungsvorrichtung aufgenommen wurde.

Das Kurzpuls-Laser-Augenchirurgiegerät weist darüber hinaus eine Vergleichsvorrichtung zum Vergleichen des mindestens einen ersten Messwerts mit einem Referenzwert zum Bestimmen des Füllstands der Flüssigkeit in dem Hohlraum auf. Die Vergleichsvorrichtung kann ein analoger Schaltkreis, ein digitaler Schaltkreis, ein integrated circuit (IC) und/oder ein Computer sein. Die Vergleichsvorrichtung kann auch zusammen mit der Bestimmungsvorrichtung ausgebildet sein. Vorstellbar ist auch, dass ein einziger Computer oder ein einziger integrated circuit sowohl die Bestimmungsvorrichtung als auch die Vergleichsvorrichtung ist. Der Vergleich kann z.B. durch Differenzbildung und/oder Verhältnisbildung zwischen dem mindestens einen ersten Messwert und dem Referenzwert durchgeführt werden. Wenn mehrere erste Messwerte vorliegen, kann jeder einzelne erste Messwert mit dem Referenzwert verglichen werden. Die Vergleichsvorrichtung kann durch den Vergleich feststellen, dass sich der mindestens eine erste Messwert (bei mehreren ersten Messwerten z.B. zumindest einer der ersten Messwerte) von dem Referenzwert unterscheidet, insbesondere mehr als ein Toleranzwert bzw. Schwellenwert unterscheidet, d.h. dass der erste Messwert außerhalb eines Toleranzbereichs um den Referenzwert herum liegt. Dies bedeutet, dass der Unterschied größer als ein Toleranzwert ist. Wenn sich der erste Messwert von dem Referenzwert unterscheidet, kann die Vergleichsvorrichtung feststellen, dass der Hohlraum nicht im Wesentlichen vollständig mit Flüssigkeit gefüllt ist.

Der Referenzwert ist ein fest vorgegebener Referenzwert oder der Referenzwert ist ein zweiter Messwert, der bei im Wesentlichen vollständiger Füllung des durch das Auge verschlossenen Hohlraums mit der Flüssigkeit aus Reflexion und/oder Rückstreuung und/oder Transmission von optischen Messstrahlen bestimmt wurde. Der Referenzwert kann vor der Bestimmung des mindestens einen ersten Messwerts festgelegt werden bzw. sein. Der Referenzwert kann in einem ROM und/oder einem RAM gespeichert werden. Die Dimension und die Einheit des Referenzwerts können der Dimension und der Einheit des bestimmten ersten Messwerts entsprechen. Der Referenzwert kann ein Referenzbild umfassen oder sein. Der zweite Messwert kann mehrere einzelne Messwerte umfassen. Der zweite Messwert bei dem Verfahren kann ein direkt erfasster Messwert oder ein hieraus abgeleiteter (z.B. berechneter) Messwert sein. Beispielsweise kann der zweite Messwert eine optische oder geometrische Weglänge zwischen zwei Elementen bzw. Teilen des Flüssigkeits-Patienteninterfaces und/oder ein durchschnittlicher Helligkeitswert bzw. Intensitätswert eines von der Erfassungsvorrichtung aufgenommenen Bilds sein. Der zweite Messwert kann auch mehrere zweite Messwerte umfassen. Der zweite Messwert kann z.B. ein 2D- oder 3D-Digitalbild sein, das von der Erfassungsvorrichtung aufgenommen wurde. Vorstellbar ist auch, dass die Vergleichsvorrichtung durch Differenzbildung zwischen dem mindestens einen ersten Messwert und dem Referenzwert feststellt, inwieweit der Hohlraum mit Flüssigkeit gefüllt ist. Dies trifft insbesondere bei einer Bestimmung der optischen Weglänge als mindestens einen ersten Messwert zu.

Vorteilhaft an diesem Kurzpuls-Laser-Augenchirurgiesystem ist, dass der Füllstand bzw. Spiegel der Flüssigkeit in dem Hohlraum des Kurzpuls-Laser-Augenchirurgiesystems in der Regel technisch einfach bestimmbar ist. Dies erhöht üblicherweise die Sicherheit der Behandlung des Auges mit dem Kurzpuls-Laser-Augenchirurgiesystem. Zudem ist das Flüssigkeits-Patienteninterface technisch einfach aufgebaut und kostengünstig herstellbar, da das Flüssigkeits-Patienteninterface in der Regel für die Bestimmung des Füllstands bzw. des Spiegels der Flüssigkeit in dem Hohlraum nicht zwingend speziell ausgebildet sein muss und keine elektrischen Kontakte aufweisen muss. Dies senkt die Kosten des Betriebs des Kurzpuls-Laser-Augenchirurgiesystems, da das Flüssigkeit-Patienteninterface nach jedem Patienten aus Sterilitätsgründen ausgetauscht werden muss. Auch lässt sich das Flüssigkeit-Patienteninterface in der Regel technisch einfach mit üblichen bekannten Verfahren sterilisieren, da das Flüssigkeit-Patienteninterface keine Metallelemente bzw. metallischen Elemente aufweist. Somit lässt sich typischerweise eine Behandlung des Auges mit dem Laserstrahl des Kurzpuls-Laser-Augenchirurgiesystems bei nicht-vollständiger Füllung des Hohlraums mit der Flüssigkeit sicher verhindern.

Die Aufgabe wird auch durch ein Verfahren zum Bestimmen eines Füllstands einer Flüssigkeit in einem Hohlraum eines Kurzpuls-Laser-Augenchirurgiesystems gelöst. Hierbei umfasst das Kurzpuls-Laser-Augenchirurgiesystem ein Kurzpuls-Laser-Augenchirurgiegerät zum Erzeugen eines Laserstrahls zur Behandlung eines Auges und ein mit dem Kurzpuls-Laser-Augenchirurgiegerät verbundenes Flüssigkeits-Patienteninterface zum Fixieren des Auges. Der Hohlraum ist in dem Flüssigkeits-Patienteninterface ausgebildet, wobei das Flüssigkeits-Patienteninterface derart mit dem Kurzpuls-Laser-Augenchirurgiegerät verbunden ist, dass der Laserstrahl durch ein Ausgangselement des Flüssigkeits-Patienteninterfaces in den Hohlraum eintritt. Der Hohlraum ist somit in Richtung des Laserstrahls dem Kurzpuls-Laser-Augenchirurgiegerät nachgeordnet.

Der Hohlraum ist auf einer dem Ausgangselement abgewandten Seite offen und von dem Auge verschließbar. Der Hohlraum kann durch Anlegen des Auges an das Flüssigkeits-Patienteninterface derart verschlossen werden, dass im verschlossenen Zustand des Hohlraums das Auge entlang der Richtung des Laserstrahls dem Flüssigkeits-Patienteninterface nachgeordnet ist.

Das Verfahren umfasst mehrere Schritte.

Ein Schritt des Verfahrens ist das Aussenden von optischen Messstrahlen aus dem Kurzpuls-Laser-Augenchirurgiegerät in das Flüssigkeits-Patienteninterface. Die optischen Messstrahlen können kohärentes Licht und/oder nicht-kohärentes Licht umfassen und gelangen aus dem Kurzpuls-Laser-Augenchirurgiegerät in das Flüssigkeits-Patienteninterface.

Ein weiterer Schritt des Verfahrens ist das Erfassen von Reflexion und/oder Rückstreuung und/oder Transmission der optischen Messstrahlen aus dem Flüssigkeits-Patienteninterface in dem Kurzpuls-Laser-Augenchirurgiegerät. Die Reflexion und/oder Rückstreuung und/oder Transmission kann mit einer Erfassungsvorrichtung erfasst werden, wie z.B. einer Kamera und/oder einem charge-coupled-device (CCD) und/oder einem optischen Sensor und/oder einem Helligkeitssensor. Die Reflexion und/oder Rückstreuung und/oder Transmission bei dem Verfahren kann sichtbares und/oder nicht-sichtbares Licht erfassen, daher kann die Erfassungsvorrichtung für sichtbares und/oder nicht-sichtbares Licht, z.B. Infrarotlicht, ausgelegt sein. Somit kann von dem Flüssigkeits-Patienteninterface und/oder von dem Auge zurückgeworfenes Licht und/oder Licht, das durch das Flüssigkeits-Patienteninterface oder einen Teil hiervon transmittiert ist, erfasst bzw. gemessen werden.

Ein weiterer Schritt des Verfahrens ist das Bestimmen mindestens eines ersten Messwerts aus der erfassten Reflexion und/oder Rückstreuung und/oder Transmission der optischen Messstrahlen. Das Bestimmen kann beispielsweise ein Berechnen umfassen. Das Bestimmen kann mittels einer Bestimmungsvorrichtung durchgeführt werden. Die Bestimmungsvorrichtung kann z.B. einen Prozessor und/oder einen integrated circuit und/oder eine analoge oder digitale Schaltung umfassen. Der mindestens eine erste Messwert kann ein direkt erfasster Wert, wie z.B. ein gemessener Strom eines Helligkeitssensors, sein. Alternativ kann der mindestens erste Messwert ein aus dem erfassten Wert bzw. Werten abgeleiteter bzw. berechneter Messwert sein. Beispielsweise kann der mindestens eine erste Messwert ein durchschnittlicher Helligkeitswert eines aufgenommenen Bilds sein. Der mindestens eine erste Messwert kann auch mehrere erste Messwerte umfassen. Der mindestens eine erste Messwert bei dem Verfahren kann z.B. ein aufgenommenes 2D- oder 3D-Digitalbild sein.

Ein weiterer Schritt des Verfahrens ist das Vergleichen des mindestens einen ersten Messwerts mit einem Referenzwert zum Bestimmen des Füllstands der Flüssigkeit in dem Hohlraum. Das Vergleichen kann durch eine Vergleichsvorrichtung durchgeführt werden, wie z.B. einem analogen Schaltkreis, einem digitalen Schaltkreis, einem integrated circuit (IC) und/oder einem Computer. Die Vergleichsvorrichtung bei dem Verfahren kann auch zusammen mit der Bestimmungsvorrichtung ausgebildet sein. Vorstellbar ist auch, dass ein Computer sowohl die Bestimmungsvorrichtung als auch die Vergleichsvorrichtung ist. Das Vergleichen kann z.B. durch Differenzbildung und/oder Verhältnisbildung zwischen dem mindestens einen ersten Messwert und dem Referenzwert durchgeführt werden. Wenn mehrere erste Messwerte vorliegen kann jeder einzelne erste Messwert mit dem Referenzwert verglichen werden. Durch das Vergleichen kann festgestellt werden, dass sich der mindestens eine erste Messwert von dem Referenzwert, insbesondere um mehr als ein Toleranzwert bzw. Schwellenwert, unterscheidet. Wenn eine Abweichung zwischen dem ersten Messwert und dem Referenzwert festgestellt wird, wird festgestellt, dass der Hohlraum nicht vollständig mit Flüssigkeit gefüllt ist. Es ist auch denkbar, dass durch Differenzbildung zwischen dem mindestens einen ersten Messwert und dem Referenzwert festgestellt wird, inwieweit der Hohlraum mit Flüssigkeit gefüllt ist. Dies trifft insbesondere bei einer Bestimmung der optischen Weglänge als mindestens einen ersten Messwert zu.

Der Referenzwert bei dem Verfahren ist ein fest vorgegebener Referenzwert oder der Referenzwert ist ein zweiter Messwert, der bei im Wesentlichen vollständiger Füllung des durch das Auge verschlossenen Hohlraums mit der Flüssigkeit aus Reflexion und/oder Rückstreuung und/oder Transmission von optischen Messstrahlen bestimmt wurde. Der Referenzwert bei dem Verfahren kann vor der Bestimmung des mindestens einen ersten Messwerts festgelegt werden. Der Referenzwert und/oder der zweite Messwert kann in einem RAM oder einem ROM gespeichert werden. Die Dimension und die Einheit des Referenzwerts können der Dimension und der Einheit des bestimmten ersten Messwerts entsprechen. Der Referenzwert bei dem Verfahren kann ein Referenzbild umfassen oder sein. Der zweite Messwert kann mehrere einzelne Messwerte umfassen. Der zweite Messwert bei dem Verfahren kann ein direkt erfasster Messwert oder ein aus einem oder mehreren erfassten Messwerten abgeleiteter Messwert sein.

Ein Vorteil dieses Verfahrens ist, dass das Verfahren typischerweise mit einem kostengünstig herstellbaren und technisch einfach ausgebildeten Flüssigkeits-Patienteninterface durchführbar ist. Das Flüssigkeits-Patienteninterface kann typischerweise technisch einfach ausgebildet und kostengünstig herstellbar sein, da das Flüssigkeits-Patienteninterface typischerweise für die Bestimmung des Füllstands der Flüssigkeit in dem Hohlraum nicht besonders ausgebildet sein muss und insbesondere keine elektrischen Anschlüsse aufweisen muss. Das Verfahren kann in der Regel mit einem Flüssigkeits-Patienteninterface durchgeführt werden, das mit üblichen bekannten Verfahren sterilisierbar ist, da das Flüssigkeits-Patienteninterface in der Regel keine Metallelemente aufweist. Dies senkt in der Regel die Kosten des Verfahrens. Zudem müssen typischerweise keine elektrischen Anschlüsse mit dem Flüssigkeits-Patienteninterface zum Bestimmen des Spiegels bzw. Füllstands der Flüssigkeit in dem Hohlraum verbunden werden. Darüber hinaus kann der Spiegel bzw. Füllstand der Flüssigkeit in dem Hohlraum in der Regel technisch einfach präzise bestimmt werden. Somit wird typischerweise eine Behandlung des Auges mit dem Laserstrahl des Kurzpuls-Laser-Augenchirurgiesystems bei nicht-vollständiger Füllung des Hohlraums mit der Flüssigkeit sicher verhindert.

Die Flüssigkeit kann insbesondere eine physiologische Flüssigkeit, z.B. eine sogenannte Balanced Salt Solution (BSS) sein. Die BSS-Lösung bzw. BSS kann insbesondere einen Brechungsindex aufweisen, der im Wesentlichen dem Brechungsindex des Auges und/oder des Ausgangselements des Flüssigkeits-Patienteninterfaces entspricht oder diesem sehr ähnlich ist. Die BSS kann einen physiologischen pH-Wert und eine isotonische Salzkonzentration aufweisen.

Die Flüssigkeit bzw. die BSS kann z.B. folgende Lösung sein:
Eine Lösung, die pro 1 ml folgendes enthält: Natriumchlorid (NaCL) 6,4 mg, Kaliumcholrid (KCl) 0,75 mg, Calciumchloriddihydrat (CaCl2•2H2O) 0,48 mg, Magnesiumchlorithexahydrat (MgCl2•6H2O) 0,3 mg, Natriumacetattrihydrat (C2H3NaO2•3H2O) 3,9 mg, Natriumcitratdihydrat (C6H5Na3O7•2H2O) 1,7 mg, Natriumhydroxid und/oder -hydrochlorsäure (zur Einstellung des pH-Werts), und Wasser zur Injektion. Der pH-Wert beträgt z.B. ca. 7,5. Die Osmolalität kann z.B. ca. 300 mOsm/kg betragen.

Jedoch sind auch andere BSS-Zusammensetzungen einsetzbar.

Die optischen Messstrahlen können insbesondere sichtbares Licht und/oder Infrarotlicht umfassen.

Die optischen Messstrahlen können insbesondere kohärentes Licht (z.B. einen Laserstrahl) und/oder inkohärentes Licht aufweisen.

Gemäß einer Ausführungsform umfasst der mindestens eine erste Messwert eine optische Weglänge zwischen dem Ausgangselement und dem Auge und der Referenzwert ist eine optische Weglänge zwischen dem Ausgangselement und dem Auge bei im Wesentlichen vollständiger Füllung des durch das Auge verschlossenen Hohlraums mit der Flüssigkeit. Vorteilhaft hieran ist, dass der Füllstand bzw. Spiegel der Flüssigkeit in dem Hohlraum typischerweise besonders genau bestimmbar ist. Somit können in der Regel auch bereits geringfügige Änderungen gegenüber einer vollständigen Füllung des Hohlraums mit Flüssigkeit detektiert werden. Dies erhöht üblicherweise die Sicherheit des Kurzpuls-Laser-Augenchirurgiegeräts noch weiter. Auch kann die Position des Auges relativ zu dem Flüssigkeits-Patienteninterface bzw. dem Kurzpuls-Laser-Augenchirurgiesystem in der Regel besonders präzise bestimmt werden.

Gemäß einer weiteren Ausführungsform umfasst der mindestens eine erste Messwert eine optische Weglänge zwischen dem Ausgangselement und einem Kontaktelement des Flüssigkeits-Patienteninterfaces zum unmittelbaren Kontaktieren des Auges, und der Referenzwert ist eine optische Weglänge zwischen dem Ausgangselement und dem Kontaktelement des Flüssigkeits-Patienteninterfaces zum unmittelbaren Kontaktieren des Auges bei im Wesentlichen vollständiger Füllung des Hohlraums mit der Flüssigkeit. Anders ausgedrückt ist hierbei eine optische Weglänge zwischen zwei Elementen des Flüssigkeits-Patienteninterfaces bestimmbar, deren physischer Abstand zueinander präzise (und besser als der physische Abstand zwischen Ausgangselement und Auge) bekannt ist. Ein Vorteil hiervon ist, dass in der Regel ein fest vorgegebener Referenzwert verwendet werden kann, so dass keine Messung zur Bestimmung des Referenzwerts durchgeführt werden muss, da die optische Weglänge zwischen dem Ausgangselement und dem Kontaktelement bei vollständiger Füllung des Hohlraums mit der Flüssigkeit berechenbar ist. Dies erhöht in der Regel die Zuverlässigkeit und Schnelligkeit der Bestimmung des Füllstands bzw. des Spiegels der Flüssigkeit. Zudem kann ein Bereich auf dem Kontaktelement präzise auf die geometrische Form des Ausgangselements ausgerichtet werden, so dass im Falle einer korrekten Füllung des Hohlraums keine Reflektion und/oder Rückstreuung auftritt. Eine nicht-korrekte Befüllung des Hohlraums ist dadurch umso sensitiver detektierbar.

Die Messung der optischen Weglänge zwischen zwei Elementen des Flüssigkeits-Patienteninterfaces ist einfacher bestimmbar, wenn das Flüssigkeits-Patienteninterface selbst bestimmte geometrische und optische Anforderungen erfüllt:

In einer Ausführungsform definieren das Ausgangselement und der Übergang zwischen Kontaktelement und Patientenauge die Weglänge zwischen zwei Elementen des Flüssigkeits-Patienteninterfaces. In diesem Fall ist es beispielsweise hilfreich, dass der Übergang vom Kontaktelement zum Auge in Richtung des Laserstrahls bzw. in einer Richtung parallel hierzu von dem Arbeitsbereich der optischen Messstrahlen liegt.

Des Weiteren sollten die das Auge treffenden und die das Kontaktelement treffenden Messstrahlen unterschiedlich reflektiert bzw. rückgestreut werden.

In einer anderen Ausführungsform definieren das Ausgangselement und der Übergang zwischen Kontaktelement und seitlichen Wand des Patienteninterfaces die optische Weglänge zwischen zwei Elementen des Flüssigkeits-Patienteninterfaces.

In diesem Fall ist es beispielsweise hilfreich, dass der Übergang vom Kontaktelement zur seitlichen Wand in Richtung des Laserstrahls bzw. in einer Richtung parallel hierzu von dem Arbeitsbereich der optischen Messstrahlen liegt. Des Weiteren sollten die das Kontaktelement treffenden und die die seitliche Wand treffenden optischen Messstrahlen in unterschiedliche Richtungen reflektiert bzw. rückgestreut werden.

In einer anderen Ausführungsform befindet sich eine Markierung bzw. ein Reflexionselement, z.B. in Form einer Kerbe, eines Vorsprungs oder in Form eines oberflächenbehandelten Teilbereichs der Innenseite der seitlichen Wand und/oder des Kontaktelements, der eine erhöhte Rauigkeit und/oder Reflektions- bzw. Rückstreueigenschaften aufweist. In diesem Fall ist es beispielsweise hilfreich, dass die Markierung in Richtung des Laserstrahls bzw. in einer Richtung parallel hierzu von dem Arbeitsbereich der optischen Messstrahlen liegt.

Des Weiteren sollten die die Markierung bzw. das Reflexionselement treffenden und die die seitliche Wand treffenden optischen Messstrahlen in unterschiedliche Richtungen reflektiert bzw. rückgestreut werden. Das Reflexionselement und das Ausgangselement (z.B. eine konvexe Linse) können den gleichen Mittelpunkt aufweisen. Das Reflexionselement kann derart ausgebildet sein, dass der Unterschied zwischen einem im Wesentlichen vollständig gefüllten Hohlraum mit Flüssigkeit und einem nicht-vollständig gefüllten Hohlraum sehr groß ist, so dass dies technisch einfach und auch bereits bei geringer Abweichung von dem Zustand der im Wesentlichen vollständigen Füllung des Hohlraums mit Flüssigkeit detektiert werden kann. Beispielsweise können bei dem Zustand, bei dem der Hohlraum im Wesentlichen vollständig mit Flüssigkeit gefüllt ist, im Wesentlichen keine der optischen Messstrahlen reflektiert/rückgestreut werden und bei dem Zustand, bei dem der Hohlraum nicht vollständig mit Flüssigkeit gefüllt ist, über 10%, 20%, 25%, 50% oder mehr als 75% der optischen Messstrahlen reflektiert/rückgestreut werden.

Gemäß einer Ausführungsform umfasst der mindestens eine erste Messwert ein Intensitätssignal, wobei das Intensitätssignal eine Intensität der Transmission der ausgesandten optischen Messstrahlen durch das Ausgangselement angibt, und wobei der Referenzwert ein Intensitätssignal der Transmission der ausgesandten optischen Messstrahlen durch das Ausgangselement bei im Wesentlichen vollständiger Füllung des Hohlraums mit der Flüssigkeit ist. Hierdurch lässt sich technisch einfach der Füllstand der Flüssigkeit in dem Hohlraum bestimmen. Die optischen Messstrahlen, die von dem Kurzpuls-Laser-Augenchirurgiegerät ausgesandt werden, können auf einer ersten Seite in das Ausgangselement eingeleitet werden, das Ausgangselement durchlaufen, aus dem Ausgangselement auf einer zweiten Seite austreten und anschließend wieder in das Kurzpuls-Laser-Augenchirurgiegerät gelangen (Transmission). Bei dieser Ausführungsform werden die optischen Messstrahlen durch interne Totalreflexion innerhalb des Ausgangselements mehrfach reflektiert und setzen sich entlang der Erstreckungsrichtung des Ausgangselements in dem Ausgangselement fort, wenn der Hohlraum nicht vollständig mit Flüssigkeit gefüllt ist bzw. das Ausgangselement auf der dem Hohlraum zugewandten Seite nicht mit Flüssigkeit benetzt ist. Bei dieser Ausführungsform sinkt das Intensitätssignal der Transmission, je größer die Fläche des Ausgangselements ist, die mit Flüssigkeit benetzt ist, da die optischen Messstrahlen an den mit Flüssigkeit benetzten bzw. bedeckten Stellen des Ausgangselements aus dem Ausgangselement gelangen (da hier ein geringer Unterschied in den Brechungsindizes zwischen dem Ausgangselement und dem Medium in dem Hohlraum vorhanden ist). Das Ausgangselement oder das Ausgangselement und ein Teil der seitlichen Wand des Flüssigkeits-Patienteninterfaces können als Wellenleiter verwendet werden. Bei der Bestimmung des Referenzwerts kann der Hohlraum nicht nur bis zu einer Marke mit Flüssigkeit gefüllt sein, sondern derart vollständig mit Flüssigkeit gefüllt sein, dass überhaupt keine Luft mehr im Hohlraum vorhanden ist.

Gemäß einer weiteren Ausführungsform umfassen die Strahlerzeugungsvorrichtung und/oder die Erfassungsvorrichtung ein Kohärenztomografiegerät zur optischen Kohärenztomografie und/oder eine Scheimpflug-Kamera. Hierdurch kann der Spiegel der Flüssigkeit in dem Hohlraum in der Regel besonders zuverlässig, exakt und technisch einfach bestimmt werden. Darüber hinaus kann hierdurch in der Regel gleichzeitig mit der Bestimmung des Spiegels der Flüssigkeit in dem Hohlraum auch die genaue Position und/oder Form des Auges und seiner Strukturen bestimmt werden.

Gemäß einer weiteren Ausführungsform umfasst der mindestens eine erste Messwert ein Intensitätssignal, wobei das Intensitätssignal eine Intensität der Rückstreuung und/oder Reflexion der ausgesandten optischen Messstrahlen angibt. Vorteilhaft hieran ist, dass die Bestimmungsvorrichtung und/oder die Vergleichsvorrichtung in der Regel technisch besonders einfach ausgebildet sein können, da keine aufwendigen und/oder komplizierten Berechnungen durchgeführt werden müssen.

Gemäß einer weiteren Ausführungsform umfasst die Erfassungsvorrichtung eine visuelle Kamera und/oder eine Infrarotkamera zum Detektieren von Reflexion und/oder Rückstreuung und/oder Transmission der optischen Messstrahlen. Ein Vorteil hiervon ist, dass das Kurzpuls-Laser-Augenchirurgiesystem in der Regel technisch einfach und kostengünstig ausgebildet ist, da visuelle Kameras und/oder Infrarotkameras kostengünstig sind. Auch kann typischerweise bei einem nichtgleichmäßigen Spiegel bzw. Füllstand der Flüssigkeit in dem Hohlraum der Spiegel der Flüssigkeit in dem Hohlraum bzw. die nicht-vollständige Füllung des Hohlraums mit der Flüssigkeit sicher erkannt werden.

Gemäß einer weiteren Ausführungsform weist die Bestimmungsvorrichtung eine Bildanalysevorrichtung zum Analysieren eines von der visuellen Kamera und/oder Infrarotkamera aufgenommen Bilds zum Erzeugen des mindestens einen ersten Messwerts auf. Ein Vorteil hiervon ist, dass typischerweise aus den von der visuellen Kamera und/oder Infrarotkamera erfassten optischen Messstrahlen technisch einfach der mindestens eine erste Messwert bestimmbar ist, z.B. durch eine Bild- oder Videoaufnahme. Dies erleichtert üblicherweise den Vergleich mit dem Referenzwert. Darüber hinaus ist vorteilhaft, dass die optischen Messstrahlen nicht kohärent sein müssen. Dies macht das Bildgebungs- und Bildanalyseverfahren technisch einfach und senkt damit die Herstellungskosten der Strahlerzeugungsvorrichtung. Der Bildvergleich zwischen erstem und zweitem Messwert kann dann durch Bildanalyse mittels Bildverarbeitungsalgorithmus erfolgen. Dieser kann in einer vorteilhaften, einfachen Ausführung durch Differenzbildung der Grauwerte oder Helligkeitswerte erfolgen. Falls der erste Messwert und der zweite Messwert im Wesentlichen identisch sind (weil sich am Füllstand der Flüssigkeit in dem Hohlraum zwischen dem zweiten Zeitpunkt, an dem der erste Messwert gemessen wird, und dem ersten Zeitpunkt, an dem der zweite Messwert gemessen wird, nichts geändert hat), erhält man nach der Differenzbildung ein Schwarzbild, d.h. alle Bildpixel haben den Grauwert oder den Helligkeitswert Null (ohne optisches Rauschen). Sobald sich jedoch die beiden Messwerte unterscheiden, gibt es Abweichungen vom Schwarzbild, die wiederum auf eine veränderte optische Abbildung, z.B. aufgrund vollständiger oder teilweiser Abwesenheit von BSS, schließen lassen. Auf diese Art und Weise ist eine zweidimensionale Überwachung des gesamten optischen Arbeitsbereichs mit einfachen Mitteln und unmittelbarer Rückmeldung möglich: Eine Warnmeldung kann beispielsweise ergehen, wenn festgestellt wird, dass die Summe aller Bildpixel oder aber die Summe der Bildpixel eines festgelegten Bildbereichs des Differenzbilds ungleich Null oder größer als ein vorgegebener Schwellenwert bzw. Toleranzwert (da optisches Rauschen auftreten kann) ist, ggf. kann in Abhängigkeit von der Summe der Bildpixel automatisch reagiert werden, z.B. der Laserstrahl abgeschaltet werden. Gleichzeitig kann sofort örtlich aufgelöst werden, an welchen Stellen der Füllstand der Flüssigkeit nicht mehr ausreichend ist.

Gemäß einer weiteren Ausführungsform umfasst der mindestens eine Messwert ein zu einem zweiten Zeitpunkt aufgenommenes Bild von Intensitätswerten und der Referenzwert umfasst ein zu einem ersten Zeitpunkt aufgenommenes Bild von Intensitätswerten, wobei die Vergleichsvorrichtung zum Vergleichen des mindestens einen ersten Messwerts mit dem Referenzwert das zu dem ersten Zeitpunkt aufgenommene Bild von dem zu dem zweiten Zeitpunkt aufgenommenen Bild subtrahiert, wobei die Intensitätswerte jeweils einer Intensität der Rückstreuung und/oder Reflexion der ausgesandten optischen Messstrahlen entsprechen. Die Intensitätswerte des aufgenommenen Bilds können die Intensität des sichtbaren und/oder nicht-sichtbaren Lichts an jeweils unterschiedlichen Punkten bzw. Stellen angeben bzw. widerspiegeln. Durch Subtraktion bzw. Differenzbildung bzw. Bildung eines Differenzbilds kann ein im Wesentlichen schwarzes Bild bzw. Bild mit Nullwerten entstehen, wenn der Füllstand der Flüssigkeit zu dem ersten Zeitpunkt und zu dem zweiten Zeitpunkt gleich ist. Wenn der Hohlraum zu dem ersten Zeitpunkt im Wesentlichen vollständig mit Flüssigkeit gefüllt ist, kann sich somit technisch einfach feststellen lassen, ob der Hohlraum zu dem zweiten Zeitpunkt (immer noch) vollständig mit Flüssigkeit gefüllt ist. Wenn nach Subtraktion bzw. Differenzbildung Stellen bzw. Bereiche mit Werten ungleich Null vorhanden sind, kann dies bedeuten, dass an den entsprechenden Stellen bzw. Bereichen der Hohlraum nicht (mehr) vollständig mit Flüssigkeit gefüllt ist. Ein Vorteil hiervon ist, dass technisch einfach mittels einer Kamera ein Entweichen von Flüssigkeit aus dem Hohlraum festgestellt werden kann.

Gemäß einer weiteren Ausführungsform des Verfahrens ist der Referenzwert eine optische Weglänge zwischen dem Ausgangselement des Flüssigkeits-Patienteninterfaces und dem den Hohlraum verschließenden Auge zu einem ersten Zeitpunkt, an dem der Hohlraum vollständig mit der Flüssigkeit gefüllt ist, und der mindestens eine erste Messwert umfasst eine optische Weglänge zwischen dem Ausgangselement des Flüssigkeits-Patienteninterfaces und dem den Hohlraum verschließenden Auge zu einem zweiten Zeitpunkt, an dem der Füllstand der Flüssigkeit in dem Hohlraum bestimmt wird. Vorteilhaft hieran ist, dass der Füllstand bzw. Spiegel der Flüssigkeit in dem Hohlraum in der Regel sehr genau bestimmt wird. Durch das Verfahren werden typischerweise auch bereits geringe Änderungen des Füllstands des Hohlraums gegenüber einer vollständigen Füllung des Hohlraums mit Flüssigkeit erkannt. Dies erhöht in der Regel die Sicherheit des Kurzpuls-Laser-Augenchirurgiegeräts. Zudem kann durch das Verfahren auch die Position des Auges in der Regel besonders exakt bestimmt werden.

Da sich das Auge des Patienten üblicherweise in Richtung des Erdbodens dem Flüssigkeits-Patienteninterface nachgeordnet befindet, steigen eventuelle Luftblasen innerhalb der Flüssigkeit in dem Hohlraum in Richtung des Ausgangselements auf. Wenn das Ausgangselement ein optisches Element ist, d.h. eine Wölbung bzw. Biegung aufweist, sammeln sich Luftblasen an der Stelle bzw. den Stellen des Ausgangselements, an der bzw. an denen das Ausgangselement den kleinsten Abstand zu dem Kurzpuls-Laser-Augenchirurgiegerät aufweist bzw. den größten Abstand vom Erdboden hat.

Der optimale Bereich, an dem die Messwerte aufgenommen werden können, wird wesentlich durch die Form des Ausgangselements bestimmt. Im Folgenden wird davon ausgegangen, dass das Auge des Patienten zwischen dem Kurzpuls-Laser-Augenchirurgiegerät und dem Erdboden angeordnet ist, so dass die Richtung der Gravitation im Wesentlichen in Richtung des Laserstrahls verläuft. Im Falle eines flachen Ausgangselements, können die Messwerte grundsätzlich an jeder beliebigen lateralen Position innerhalb eines Arbeitsbereichs der optischen Messstrahlen, d.h. über die komplette Breite des Hohlraums, bzw. des Lasers, d.h. z.B. innerhalb des Kontaktelements, bestimmt werden. Ist das Ausgangselement hingegen konvex, so ist die ideale Position zur Messwertbestimmung am seitlich äußeren Rand des Hohlraums, wo der Abstand zwischen Ausgangselement und Erdboden größer ist als entlang der Mittellinie des Hohlraums. Die Mittellinie des Hohlraums verläuft in Richtung der Gravitationskraft durch die Mitte des Hohlraums. An der Mittellinie des Hohlraums wird die Flüssigkeit den Hohlraum zwischen Hornhaut bzw. Auge und Ausgangselement zuerst vollständig füllen, da hier der physische Abstand zwischen Ausgangselement und Auge am kleinsten ist. Ist das Ausgangselement konkav gestaltet, werden die seitlich äußeren Bereiche des Hohlraums, die von der Mittellinie am weitesten entfernt sind, zuerst mit der Flüssigkeit gefüllt sein und entlang der Mittellinie des Hohlraums zuletzt. Daher wird in diesem Falle der Messwert idealerweise entlang der Mittellinie oder nahe der Mittellinie des Hohlraums bestimmt.

Gemäß einer weiteren Ausführungsform des Verfahrens umfasst der mindestens eine erste Messwert eine optische Weglänge zwischen dem Ausgangselement des Flüssigkeits-Patienteninterfaces und einem Kontaktelement des Flüssigkeits-Patienteninterfaces zum unmittelbaren Kontaktieren des Auges. Mit anderen Worten wird hierbei eine optische Weglänge zwischen zwei Elementen des Flüssigkeits-Patienteninterfaces bestimmt, deren physischer Abstand bekannt ist. Ein Vorteil hiervon ist, dass bei dem Verfahren typischerweise ein fest vorgegebener Referenzwert verwendet werden kann. Somit muss bei diesem Verfahren in der Regel keine Messung zur Bestimmung des Referenzwerts durchgeführt werden, da die optische Weglänge zwischen dem Ausgangselement und dem Kontaktelement bei vollständiger Füllung des Hohlraums mit der Flüssigkeit berechenbar ist. Dies erhöht typischerweise die Zuverlässigkeit und Schnelligkeit des Verfahrens.

Gemäß einer weiteren Ausführungsform wird die optische Weglänge mittels optischer Kohärenztomografie und/oder mittels einer Messung mit einer Scheimpflug-Kamera bestimmt. Vorteilhaft hieran ist, dass üblicherweise technisch einfach und mit hoher Präzision der Spiegel bzw. Füllstand der Flüssigkeit in dem Hohlraum bestimmt wird. Darüber hinaus kann hierdurch in der Regel gleichzeitig mit der Bestimmung des Spiegels der Flüssigkeit in dem Hohlraum auch die genaue Position und/oder Form des Auges bestimmt werden.

Gemäß einer weiteren Ausführungsform umfasst das Ausgangselement eine Ausgangslinse und die optische Weglänge wird entlang der optischen Achse der Ausgangslinse und/oder parallel zu einer optischen Achse des Kurzpuls-Laser-Augenchirurgiegeräts gemessen. Hierdurch wird der Spiegel bzw. Füllstand der Flüssigkeit in dem Hohlraum typischerweise besonders genau bestimmt, da keine Ablenkung der ausgesandten optischen Messstrahlen durch die Ausgangslinse stattfindet.

Bei einem nicht-planaren bzw. gewölbten Ausgangselement kann die optische Weglänge in der Nähe bzw. unmittelbar benachbart dem äußeren Rand des Ausgangselements bestimmt werden.

Gemäß einer weiteren Ausführungsform des Verfahrens umfasst der mindestens eine erste Messwert ein Intensitätssignal, wobei das Intensitätssignal eine Intensität der Transmission der ausgesandten optischen Messstrahlen durch das Ausgangselement angibt, und wobei der Referenzwert ein Intensitätssignal der Transmission der ausgesandten optischen Messstrahlen durch das Ausgangselement bei im Wesentlichen vollständiger Füllung des Hohlraums mit der Flüssigkeit ist. Hierdurch lässt sich der Füllstand der Flüssigkeit in dem Hohlraum technisch einfach bestimmen.

Gemäß einer weiteren Ausführungsform des Verfahrens umfassen die erfasste Reflexion und/oder Rückstreuung Reflexion und/oder Rückstreuung der optischen Messstrahlen des den Hohlraum verschließenden Auges und/oder des Kontaktelements. Hierdurch wird typischerweise sichergestellt, dass bei dem Verfahren nicht nur Rückstreuung und/oder Reflexion an der Grenzfläche zwischen dem Ausgangselement und dem Hohlraum und/oder an der Oberfläche der Flüssigkeit bei der Bestimmung des Füllstands bzw. Spiegels der Flüssigkeit in dem Hohlraum berücksichtigt wird, sondern auch Rückstreuung und/oder Reflexion an der Grenzfläche zwischen dem Hohlraum und dem Auge berücksichtigt wird. Dies erhöht in der Regel die Sicherheit des Kurzpuls-Laser-Augenchirurgiesystems weiter.

Gemäß einer weiteren Ausführungsform des Verfahrens umfasst der mindestens eine erste Messwert ein Intensitätssignal, wobei das Intensitätssignal eine Intensität der Rückstreuung und/oder Reflexion der ausgesandten optischen Messstrahlen angibt. Ein Vorteil dieses Verfahrens ist, dass die Bestimmungsvorrichtung und/oder die Vergleichsvorrichtung in der Regel technisch besonders einfach und kostengünstig ausgebildet sein können, da keine aufwendigen und/oder komplizierten Berechnungen durchgeführt werden. Somit kann das Verfahren typischerweise mit einem besonders kostengünstigen Kurzpuls-Laser-Augenchirurgiesystem durchgeführt werden. Zudem kann das Verfahren in der Regel besonders schnell durchgeführt werden.

Gemäß einer weiteren Ausführungsform wird bei dem Bestimmen des mindestens einen Messwerts eine optische Bildaufnahme des Hohlraums analysiert. Vorteilhaft hieran ist, dass in der Regel auch unterschiedliche Füllstände bzw. Spiegel der Flüssigkeit in dem Hohlraum in unterschiedlichen Teilbereichen des Hohlraums bestimmt bzw. erkannt werden können. Dies erhöht typischerweise die Sicherheit des Kurzpuls-Laser-Augenchirurgiesystems noch weiter.

Das Bestimmen des Spiegels der Flüssigkeit in dem Hohlraum kann insbesondere das Bestimmen, ob der Hohlraum vollständig mit der Flüssigkeit gefüllt ist, umfassen.

Die Reflexion und/oder Rückstreuung der optischen Messstrahlen kann insbesondere an der Grenzfläche zwischen dem Ausgangselement des Patienteninterfaces und dem Hohlraum, an der Oberfläche der Flüssigkeit bzw. dem Übergang zwischen Luft und der Flüssigkeit im Hohlraum und am Auge bzw. am Übergang zwischen Hohlraum und Auge stattfinden.

Die Messstrahlen können insbesondere optische Strahlen einer optischen Kohärenztomografie, einer Scheimpflug-Kamera und/oder sichtbare optische Strahlen (sichtbares Licht) und/oder Infrarotlicht umfassen.

Die Abhängigkeit des Brechungsindex' von der Wellenlänge ist im Rahmen der vorliegenden Erfindung üblicherweise vernachlässigbar gering.

Die hier beschriebenen Möglichkeiten zur Bestimmung des ersten Messwerts und/oder des zweiten Messwerts bzw. zur Festlegung des Referenzwerts bzw. zur Bestimmung des Füllstands der Flüssigkeit in dem Hohlraum können unabhängig voneinander angewandt werden. Alternativ ist auch vorstellbar, dass zwei oder mehr als zwei der beschriebenen Möglichkeiten zur Bestimmung des Füllstands der Flüssigkeit in dem Hohlraum miteinander kombiniert werden. Auf diese Weise kann eine höhere Zuverlässigkeit der Bestimmung des Füllstands der Flüssigkeit in dem Hohlraum erreicht werden. So kann z.B. zunächst der Füllstand mittels Bildanalyse bestimmt werden und, wenn hierbei eine nichtvollständige Füllung des Hohlraums mit Flüssigkeit erkannt wird, anschließend eine optische Abstandsmessung bzw. eine Bestimmung der optischen Weglänge durchgeführt werden. Aus den (beiden) mit unterschiedlichen Methoden bestimmten Füllständen bzw. Ergebnissen kann ein Mittelwert gebildet werden. Auch vorstellbar ist, dass der geringere bzw. kleinere der bestimmten Füllstände als tatsächlicher Füllstand angenommen wird, um eine größtmögliche Sicherheit zu gewährleisten.

Ebenfalls offenbart ist ein Flüssigkeits-Patienteninterface zum Fixieren eines zu behandelnden Auges. Das Flüssigkeits-Patienteninterface umfasst einen Hohlraum zum Aufnehmen einer Flüssigkeit und ist derart an einem Kurzpuls-Laser-Augenchirurgiegerät zum Erzeugen eines Laserstrahls zur Behandlung des Auges anordenbar, dass der Hohlraum entlang einer Richtung des Laserstrahls nach einem Ausgangselement des Flüssigkeits-Patienteninterfaces angeordnet ist. Der Hohlraum des Flüssigkeits-Patienteninterfaces ist auf einer von dem Ausgangselement abgewandten Seite offen und von dem Auge verschließbar. Gekennzeichnet ist das Flüssigkeits-Patienteninterface dadurch, dass der Hohlraum mindestens ein Reflexionselement umfasst, wobei das Reflexionselement derart ausgebildet und das Reflexionselement derart zu dem Ausgangselement angeordnet ist, dass bei im Wesentlichen vollständiger Füllung des Hohlraums mit Flüssigkeit von dem Reflexionselement im Wesentlichen keine optischen Messstrahlen, die aus Richtung des Ausgangselements auf das Reflexionselement fallen, in Richtung des Ausgangselements reflektiert und/oder rückgestreut werden und bei nicht-vollständiger Füllung des Hohlraums mit Flüssigkeit von dem Reflexionselement mindestens 10%, insbesondere mindestens 25%, vorzugsweise mindestens 50%, von optischen Messstrahlen, die aus Richtung des Ausgangselements auf das Reflexionselement fallen, in Richtung des Ausgangselements reflektiert und/oder rückgestreut werden.

Ein Vorteil hiervon ist, dass ein großer Unterschied zwischen dem Zustand, bei dem der Hohlraum im Wesentlichen vollständig mit Flüssigkeit gefüllt ist, und dem Zustand, bei dem der Hohlraum nicht-vollständig mit Flüssigkeit gefüllt ist, hinsichtlich der Reflexion und/oder Rückstreuung der optischen Messstrahlen vorhanden ist. Die Reflexion und/oder Rückstreuung der optischen Messstrahlen ist bei diesen beiden Zuständen bei dem Flüssigkeits-Patienteninterface stark unterschiedlich. Somit lässt sich in der Regel ein Ausfließen von Flüssigkeit aus dem Flüssigkeits-Patienteninterface technisch einfach und besonders zuverlässig feststellen.

Gemäß einer Ausführungsform des Flüssigkeit-Patienteninterfaces umfasst das mindestens eine Reflexionselement - mindestens einen in den Hohlraum hineinstehenden Vorsprung einer den Hohlraum teilweise begrenzenden seitlichen Wand und/oder eines Kontaktelements des Flüssigkeits-Patienteninterfaces zum unmittelbaren Kontaktieren des Auges und/oder - mindestens eine Vertiefung in einer den Hohlraum teilweise begrenzenden seitlichen Wand und/oder in einem Kontaktelement des Flüssigkeits-Patienteninterfaces zum unmittelbaren Kontaktieren des Auges, wobei das Reflexionselement den Hohlraum nicht-umlaufend ausgebildet ist. Somit kann sich das Reflexionselement nur in einem bestimmten Winkelbereich des Kontaktelements und/oder der seitlichen Wand des Hohlraums befinden und den Hohlraum nicht vollständig umlaufen. Vorteilhaft hieran ist, dass die optischen Messstrahlen gezielt in Richtung des Reflexionselements ausgestrahlt werden können, wodurch ein Entweichen von Flüssigkeit aus dem Hohlraum zu einem frühen Zeitpunkt bereits feststellbar ist.

Es wird zudem ein Flüssigkeits-Patientinterface offenbart, das ein erstes optisches Ablenkelement zum Ablenken von optischen Messstrahlen, die aus dem Kurzpuls-Laser-Augenchirurgiegerät in das Flüssigkeits-Patienteninterface ausgesandt werden, in das Ausgangselement derart, dass bei Nicht-Vorhandensein von Flüssigkeit in dem Hohlraum die optischen Messstrahlen in dem Ausgangselement einer internen Totalreflexion unterliegen, und ein zweites optisches Ablenkelement zum Ablenken der optischen Messstrahlen, die das Ausgangselement durchlaufen bzw. transmittiert haben, in Richtung des Kurzpuls-Laser-Augenchirurgiegeräts umfasst.

Zudem ist ein System offenbart, wobei das System ein Flüssigkeits-Patienteninterface wie vorstehend beschrieben und ein Kurzpuls-Laser-Augenchirurgiegerät zum Erzeugen eines Laserstrahls zur Behandlung des Auges umfasst. Das Flüssigkeits-Patienteninterface des Systems ist derart an dem Kurzpuls-Laser-Augenchirurgiegerät angeordnet, dass der Hohlraum entlang einer Richtung des Laserstrahls nach einem Ausgangselement des Flüssigkeits-Patienteninterfaces angeordnet ist. Das Kurzpuls-Laser-Augenchirurgiegerät des Systems umfasst -- eine Strahlerzeugungsvorrichtung zum Aussenden von optischen Messstrahlen aus dem Kurzpuls-Laser-Augenchirurgiegerät in das Flüssigkeits-Patienteninterface, -- eine Erfassungsvorrichtung zum Erfassen von Reflexion und/oder Rückstreuung und/oder Transmission der optischen Messstrahlen aus dem Flüssigkeits-Patienteninterface in dem Kurzpuls-Laser-Augenchirurgiegerät, -- eine Bestimmungsvorrichtung zum Bestimmen mindestens eines ersten Messwerts aus der erfassten Reflexion und/oder Rückstreuung und/oder Transmission der optischen Messstrahlen, und -- eine Vergleichsvorrichtung zum Vergleichen des mindestens einen ersten Messwerts mit einem Referenzwert zum Bestimmen des Füllstands der Flüssigkeit in dem Hohlraum. Der Referenzwert bei dem System ist ein fest vorgegebener Referenzwert oder der Referenzwert ist ein zweiter Messwert, der bei im Wesentlichen vollständiger Füllung des durch das Auge verschlossenen Hohlraums mit der Flüssigkeit aus Reflexion und/oder Rückstreuung und/oder Transmission von optischen Messstrahlen bestimmt wurde.

Vorteilhaft an diesem System ist, dass der Füllstand bzw. Spiegel der Flüssigkeit in dem Hohlraum des Kurzpuls-Laser-Augenchirurgiesystems in der Regel technisch einfach bestimmbar ist. Dies erhöht üblicherweise die Sicherheit der Behandlung des Auges mit dem System.

Bevorzugte Ausführungsformen ergeben sich aus den Unteransprüchen. Nachfolgend wird die Erfindung anhand von Zeichnungen von Ausführungsbeispielen näher erläutert. Hierbei zeigen

Fig. 1      eine schematische Seitenansicht einer ersten Ausführungsform des erfindungsgemäßen Kurzpuls-Laser-Augenchirurgiesystems;

Fig. 2a eine detaillierte schematische Querschnittsansicht einer ersten Ausführungsform des Flüssigkeits-Patienteninterfaces;

Fig. 2b eine detaillierte schematische Querschnittsansicht einer zweiten Ausführungsform des Flüssigkeits-Patienteninterfaces;

Fig. 3 ein schematisches Tomographiebild des Hohlraums bei vollständiger Füllung des Hohlraums mit der BSS;

Fig. 4 ein schematisches Tomographiebild des Hohlraums bei vollständiger Füllung des Hohlraums mit Luft;

Fig. 5 eine schematische Aufsicht auf das Auge durch das Flüssigkeits-Patienteninterface bei im Wesentlichen vollständiger Füllung des Hohlraums mit der BSS;

Fig. 6 eine schematische Aufsicht auf das Auge durch das Flüssigkeits-Patienteninterface bei nicht-vollständiger Füllung des Hohlraums mit der BSS;

Fig. 7 eine schematische Aufsicht auf das Auge durch das Flüssigkeits-Patienteninterface bei teilweiser Füllung des Hohlraums mit der BSS;

Fig. 8 eine schematische Querschnittsansicht des Hohlraums bei teilweiser Füllung des Hohlraums mit der BSS;

Fig. 9 eine detaillierte schematische Querschnittsansicht einer dritten Ausführungsform des Flüssigkeits-Patienteninterfaces sowie eines Teils des Kurzpuls-Laser-Augenchirurgiegeräts;

Fig. 10 eine detaillierte schematische Querschnittsansicht einer vierten Ausführungsform des Flüssigkeits-Patienteninterfaces sowie eines Teils des Kurzpuls-Laser-Augenchirurgiegeräts, wobei der Hohlraum mit Luft gefüllt ist;

Fig. 11 eine detaillierte schematische Querschnittsansicht der vierten Ausführungsform des Flüssigkeits-Patienteninterfaces sowie eines Teils des Kurzpuls-Laser-Augenchirurgiegeräts, wobei der Hohlraum vollständig mit Flüssigkeit gefüllt ist; und

Fig. 12 eine detaillierte schematische Querschnittsansicht der vierten Ausführungsform des Flüssigkeits-Patienteninterfaces sowie eines Teils des Kurzpuls-Laser-Augenchirurgiegeräts, wobei der Hohlraum teilweise mit Flüssigkeit gefüllt ist.

Bei der nachfolgenden Beschreibung werden für gleiche und gleich wirkende Teile dieselben Bezugsziffern verwendet.

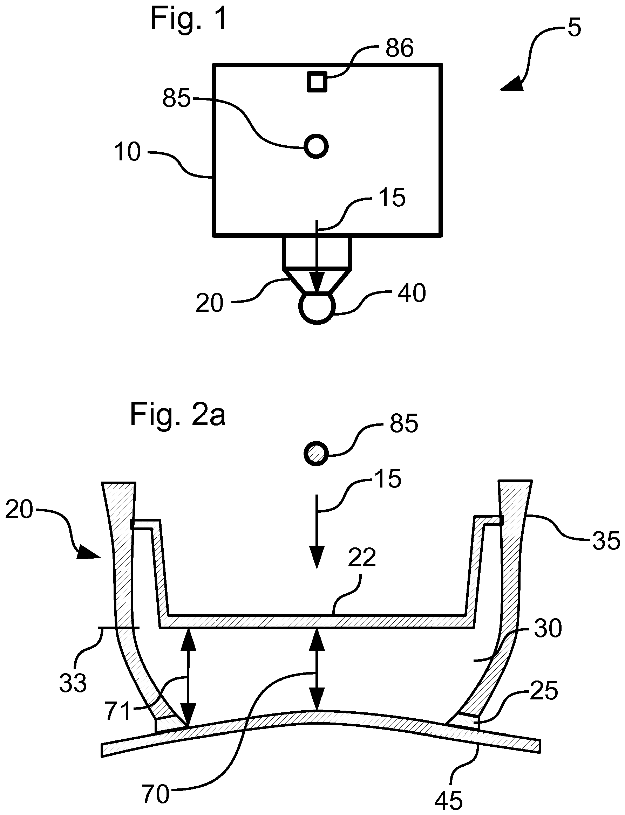

Fig. 1 zeigt eine schematische Seitenansicht einer ersten Ausführungsform des erfindungsgemäßen Kurzpuls-Laser-Augenchirurgiesystems 5. Das Kurzpuls-Laser-Augenchirurgiesystem 5 umfasst ein Kurzpuls-Laser-Augenchirurgiegerät 10 und ein Flüssigkeits-Patienteninterface 20. Das Kurzpuls-Laser-Augenchirurgiegerät 10 erzeugt einen Kurzpuls-Laserstrahl zum Behandeln des Auges 40. Insbesondere kann das Kurzpuls-Laser-Augenchirurgiegerät 10 ein Katarakt-Behandlungsgerät zum Behandeln von Katarakt (sogenannter grauer Star) eines (menschlichen oder tierischen) Auges 40 sein. Hierzu wird mittels eines Kurzpuls-Laserstrahls des Kurzpuls-Laser-Augenchirurgiegeräts 10 die natürliche (getrübte) Linse des Auges 40 in Teile geschnitten oder zerstückelt. Mittels des Laserstrahls werden ein oder mehrere Schnitte in der Hornhaut bzw. Cornea 45 und/oder Linsenkapsel des Auges 40 durchgeführt. Nun wird die natürliche Linse bzw. deren Teile entfernt. Anschließend wird eine künstliche Linse in das Auge 40 eingesetzt.

Der Laserstrahl des Kurzpuls-Laser-Augenchirurgiegeräts 10 kann Einschnitte in die Cornea, den Kapselsack der Linse und/oder in die Linse selbst durchführen.

Der Laserstrahl kann insbesondere ein Femtosekundenlaserstrahl mit einer Dauer im Bereich von ca. $10^{-13}$ s sein, z.B. 300 fs - 1000 fs, vorzugsweise 600 fs - 900 fs. Die Energie des Laserstrahlpulses liegt üblicherweise im Bereich von einigen hundert Nanojoule bis wenigen Mikrojoule. Die Wellenlänge kann z.B. im Bereich von ca. 1020 nm bis ca. 1060 nm liegen, insbesondere 1030 nm $\pm$ 5 nm sein.

Das Flüssigkeits-Patienteninterface 20 ist zwischen dem Kurzpuls-Laser-Augenchirurgiegerät 10 und dem zu behandelnden Auge 40 angeordnet. Dies bedeutet, dass in Richtung 15 des Laserstrahls, die in Fig. 1 von oben nach unten verläuft, das Flüssigkeits-Patienteninterface 20 dem Kurzpuls-Laser-Augenchirurgiegerät 10 nachgeordnet ist. Das Flüssigkeits-Patienteninterface 20 ist starr gegenüber dem Kurzpuls-Laser-Augenchirurgiegerät 10 angeordnet und dient zum Fixieren des Auges 40 relativ zu dem Kurzpuls-Laser-Augenchirurgiegerät 10 und somit relativ zum Laserstrahl des Kurzpuls-Laser-Augenchirurgiegeräts 10 bzw. zu einem Teil hiervon.

Das Flüssigkeits-Patienteninterface 20 übt nur einen geringen Druck auf das Auge 40 aus, so dass der Augeninnendruck ebenfalls gering bleibt und folglich die Retina nicht beschädigt wird. Der innere Aufbau bzw. die verschiedenen Elemente des Kurzpuls-Laser-Augenchirurgiegeräts 10 sind nicht dargestellt.

Das Auge 40 befindet sich üblicherweise in Gra-vitationsrichtung unterhalb des Flüssigkeits-Patientenin-

terfaces 20, so dass die Flüssigkeit aus dem Hohlraum 30 ausströmt, wenn das Auge die Öffnung des Hohlraums 30 nicht verschließt.

Fig. 2a zeigt eine detaillierte schematische Querschnittsansicht des Flüssigkeits-Patienteninterfaces 20. Das Flüssigkeits-Patienteninterface 20 umfasst einen Hohlraum 30. Der Hohlraum 30 (auch Zwischenraum genannt) dient zum Aufnehmen einer Flüssigkeit. Der Hohlraum 30 wird auf seiner Oberseite (in Fig. 2a oben), die dem Kurzpuls-Laser-Augenchirurgiegerät 10 zugewandt ist, durch ein Ausgangselement 22 des Flüssigkeits-Patienteninterfaces 20 begrenzt. Der Laserstrahl aus dem Kurzpuls-Laser-Augenchirurgiegerät 10 dringt durch das Ausgangselement 22 in den Hohlraum 30 ein. Das Ausgangselement 22 begrenzt den Hohlraum 30 auf der dem Auge 40 abgewandten Seite (in Fig. 2a die obere Seite des Hohlraums 30). Das Ausgangselement 22 ist für den Laserstrahl zur Behandlung des Auges 40 durchsichtig. Auch für die optischen Messstrahlen ist das Ausgangselement 22 durchsichtig. Das Ausgangselement 22 kann ein flach bzw. eben ausgebildetes Element sein. Das Ausgangselement 22 kann alternativ eine (gewölbte bzw. konvexe oder konkave) Ausgangslinse sein. An den Seiten wird der Hohlraum 30 durch eine seitliche Wand 35 des Flüssigkeits-Patienteninterface 20 begrenzt. Nach unten hin in Fig. 2a, d.h. auf der dem Ausgangselement 22 bzw. dem Kurzpuls-Laser-Augenchirurgiegerät 10 abgewandten Seite ist der Hohlraum 30 offen. Diese Öffnung des Hohlraums 30 ist durch das Auge 40 verschließbar.

Das Flüssigkeits-Patienteninterface 20 weist ein Kontaktelement 25 auf, das zum unmittelbaren Kontaktieren des Auges 40 bzw. der Cornea 45 ausgebildet sein. Das Kontaktelement 25 kann eine sogenannte Weichkomponente sein, d.h. aus einem weichen bzw. flexiblen Material ausgebildet sein. Das Kontaktelement 25 bildet gleichzeitig das Abdichtungselement zum Abdichten des Zwischenraums zwischen der Wand 35 des Flüssigkeits-Patienteninterfaces 20 und dem Auge 40 bzw. der Cornea 45. Das Kontaktelement 25 kann torusförmig ausgebildet sein. Das Kontaktelement 25 kann einen Saugring mit entsprechenden Anschlüssen zur Herstellung eines Unterdrucks in dem Hohlraum 30 umfassen.

Der Hohlraum 30 ist auf der Seite, die von dem Ausgangselement 22 abgewandt ist (in Fig. 2a die untere Seite), offen. Die Öffnung des Hohlraums 30 kann durch Anlegen des Auges 40 an das Flüssigkeits-Patienteninterface 20 verschlossen werden, wie dies in Fig. 2a gezeigt ist. In der in Fig. 2a gezeigten Position des Auges 40 ist der Hohlraum 30 flüssigkeitsdicht verschlossen.

Der Hohlraum 30 wird mit einer Flüssigkeit (z.B. einer sterilen balanced salt solution, BSS) gefüllt. Das Flüssigkeits-Patienteninterface 20 weist zum Befüllen des Hohlraums 30 eine oder mehrere verschließbare Öffnungen (nicht gezeigt) auf. Die Flüssigkeit dient zum Benetzen bzw. Befeuchten des Auges 40 bzw. eines Teils hiervon und zum Nivellieren bzw. Angleichen des Brechungsindexes des Ausgangselements 22 bzw. der Ausgangslinse des Patienteninterfaces an den Brechungsindex des Auges 40. Die Flüssigkeit hat somit einen ähnlichen Brechungsindex wie das Auge 40 und/oder wie das Ausgangselement 22. Bei vollständiger Füllung des Hohlraums 30 mit der Flüssigkeit wird somit nur ein (sehr) geringer Teil oder gegen Null gehender Teil des Laserstrahls des Kurzpuls-Laser-Augenchirurgiegeräts 10 und/oder der Messstrahlen des Kurzpuls-Laser-Augenchirurgiegeräts 10 von dem Flüssigkeits-Patienteninterface 20 und/oder dem Auge 40 bzw. der Linse 41 des Auges 40 reflektiert und/oder rückgestreut.

Das Flüssigkeits-Patienteninterface 20 arretiert bzw. fixiert das Auge 40 relativ zu dem Flüssigkeits-Patienteninterface 20 und somit relativ zu der Richtung 15 bzw. dem Fokus des Laserstrahls zum Behandeln des Auges 40. Ein absichtliches oder unabsichtliches Bewegen des Auges 40 relativ zu dem Flüssigkeits-Patienteninterface 20 wird hierdurch vermieden.

Zuleitungen zum Einbringen der Flüssigkeit in den Hohlraum 30 sind in den Zeichnungen nicht gezeigt. Auch Vorrichtungen bzw. Leitungen zum Erzeugen des Unterdrucks in dem Hohlraum 30 sind in den Zeichnungen nicht gezeigt. Ein Verlust an Flüssigkeit in dem Hohlraum 30 ist auch dadurch möglich, dass die Vorrichtung bzw. die Leitungen zum Erzeugen des Unterdrucks in dem Hohlraum 30 Flüssigkeit aus dem Hohlraum 30 saugen.

Bevorzugt ist der Hohlraum 30 im Wesentlichen trichterförmig mit einer von dem Ausgangselement 22 weg verjüngenden Form. Der Hohlraum 30 ist durch die seitliche Wand 35, dem Ausgangselement 22 und dem Auge 40 begrenzt.

Das Kurzpuls-Laser-Augenchirurgiesystem 5 überwacht bzw. bestimmt den Spiegel bzw. Füllstand der Flüssigkeit in dem Hohlraum 30. Der Spiegel (engl. level) bzw. Füllstand der Flüssigkeit gibt an, inwieweit bzw. inwiefern der Hohlraum 30 mit der Flüssigkeit gefüllt ist, insbesondere ob der Hohlraum 30 von dem Auge 40 nach oben bis zu der Marke 33 gefüllt ist. Wenn der Hohlraum 30 bis zur Marke 33 gefüllt ist, ist der Hohlraum 30 derart weit mit Flüssigkeit gefüllt, dass über den kompletten Arbeitsbereich des Laserstrahls 15, der sich zwischen den Stellen erstreckt, an denen sich in einer Richtung parallel zum Laserstrahl unterhalb des Ausgangselements 22 das Auge 40 befindet, Flüssigkeit zwischen dem Ausgangselement 22 und dem Auge 40 vorhanden ist. Die Grenzen des Arbeitsbereichs stellen somit die Stellen dar, an denen sich in einer Richtung parallel zum Laserstrahl unterhalb des Ausgangselements 22 das Kontaktelement 25 oder die seitliche Wand 35 befindet.

Wenn festgestellt wird, dass der Füllstand des Hohlraums 30 mit der Flüssigkeit eine kritische Grenze unterschreitet bzw. unterschritten hat, wird ein Alarmsignal/Warnsignal bzw. eine Warnmeldung ausgegeben und/oder der Laserstrahl unterbrochen bzw. das Einschalten des Laserstrahls verhindert. Dies erhöht die Sicherheit der Behandlung des Auges mit dem Laserstrahl.

Die kritische Grenze des Füllstands ist durch die resultierende optische Wirkung definiert. Ist der optische Arbeitsbereich des Laserstrahls 15 zwischen Ausgangselement 22 und Hornhaut/Cornea 45 nicht vollständig gefüllt, z.B. nicht bis zur Marke 33, kann die Behandlung des Auges mit dem Kurzpuls-Laser-Augenchirurgiegerät nicht sicher durchgeführt werden. Insbesondere kann ein Warnsignal ausgegeben werden und/oder der Laserstrahl unterbrochen werden bzw. das Einschalten des Laserstrahls verhindert werden, sobald festgestellt wird, dass der Hohlraum 30 nicht vollständig bzw. nicht bis zur Marke 33 mit Flüssigkeit gefüllt ist.

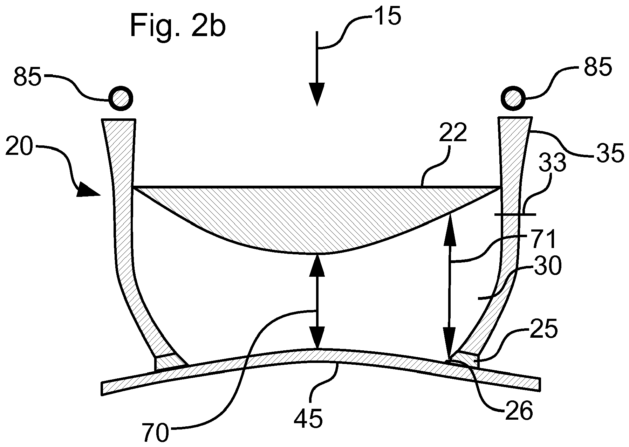

Fig. 2b zeigt eine detaillierte schematische Querschnittsansicht einer zweiten Ausführungsform des Flüssigkeits-Patienteninterfaces 20. Die in Fig. 2b gezeigte Ausführungsform unterscheidet sich von der in Fig. 2a gezeigten Ausführungsform dadurch, dass das Ausgangselement 22 nicht im Wesentlichen (in seiner Mitte) planar ausgebildet ist, sondern eine konvexe Form hat bzw. eine konvexe Linse ist. Zudem unterscheidet sich die in Fig. 2b gezeigte Ausführungsform von der in Fig. 2a gezeigten Ausführungsform dadurch, dass das Kontaktelement 25 ein Reflexionselement 26 aufweist. Das Reflexionselement 26 muss den Hohlraum 30 nicht bzw. nicht vollständig umlaufen, sondern kann sich nur in einem Winkelbereich befinden, z.B. in einem Winkelbereich von ca. 10°. Das Reflexionselement 26 ist derart ausgebildet und angeordnet, dass bei im Wesentlichen vollständiger Füllung des Hohlraums 30 mit Flüssigkeit, im Wesentlichen keine optischen Messstrahlen (die in Richtung des Laserstrahls 15 bzw. parallel hierzu auf das Reflexionselement 26 treffen) in Richtung des Ausgangselements 22 bzw. entgegen der Richtung des Laserstrahls 15 bzw. parallel hierzu in das Kurzpuls-Laser-Augenchirurgiegerät 10 reflektiert und/oder rückgestreut werden. Wenn der Hohlraum 30 nicht vollständig mit Flüssigkeit oder gar nicht mit Flüssigkeit gefüllt ist, wird ein besonders großer Teil der optischen Messstrahlen (aus dem Kurzpuls-Laser-Augenchirurgiegerät 10), die auf das Reflexionselement 26 treffen, von dem Reflexionselement 26 in Richtung des Ausgangselements 22 bzw. entgegen der Richtung des Laserstrahls 15 (in das Kurzpuls-Laser-Augenchirurgiegerät 10) reflektiert und/oder rückgestreut. Somit ist der Unterschied zwischen dem vollständig mit Flüssigkeit gefüllten Zustand des Hohlraums 30 und dem nicht-vollständig mit Flüssigkeit gefüllten Hohlraums 30 besonders einfach feststellbar. Das Reflexionselement 26 reflektiert bzw. streut die optischen Messstrahlen in anderen Winkeln zurück als die seitliche Wand des Hohlraums 30.

Das Reflexionselement 26 kann eine Vertiefung bzw. Kerbe oder ein Vorsprung sein. Das Reflexionselement 26 kann auf seiner dem Ausgangselement 22 zugewandten Seite eine gebogene bzw. gekrümmte Oberfläche aufweisen. Der Mittelpunkt des Ausgangselements 22 bzw. der konvexen Linse entspricht dem Mittelpunkt des Reflexionselements 26. Alternativ ist auch vorstellbar, dass die dem Ausgangselement 22 zugewandte Seite im Wesentlichen senkrecht zu dem Laserstrahl verläuft. Die optische Weglänge 71 kann beispielsweise an der Stelle des Reflexionselements 26 gemessen werden.

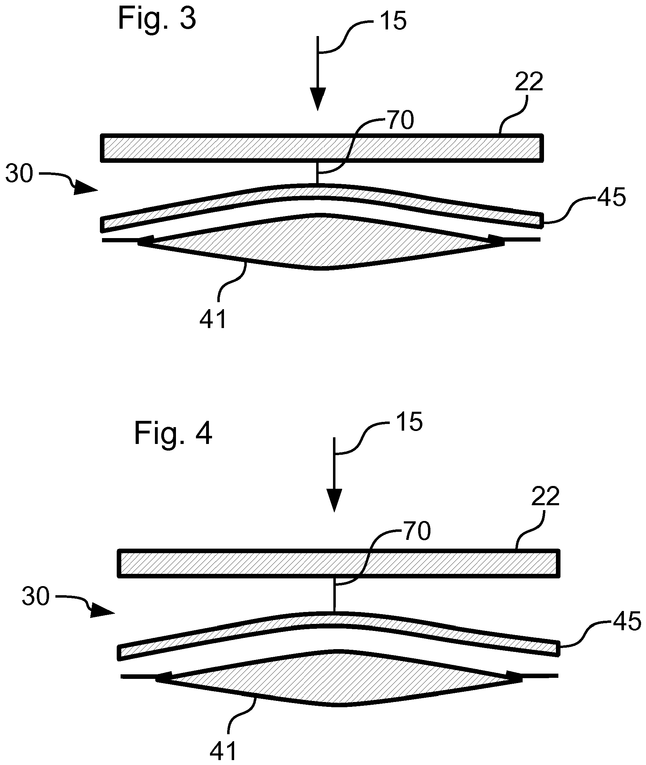

Fig. 3 zeigt ein schematisches Tomographiebild des Hohlraums 30 bei vollständiger Füllung des Hohlraums 30 mit BSS, wie es z.B. mittels Kohärenztomographie oder Scheinpflug-Bildgebung aufgenommen werden könnte. Fig. 4 zeigt ebenfalls ein schematisches Tomographiebild des gleichen Hohlraums 30 bei vollständiger Füllung des Hohlraums 30 mit Luft, d.h. wenn im Wesentlichen keine BSS im Hohlraum 30 vorhanden ist. Durch den höheren Brechungsindex der BSS gegenüber Luft erscheint der Abstand 70 zwischen Hornhaut 45 und Ausgangselement 22 geringer im Vergleich zu Fig. 3. Dies ist jedoch ein rein optischer Effekt. Der Abstand 70 wird in der Optik als optische Weglänge s bzw. optische Weglänge 71, 72 bezeichnet und ergibt sich aus dem Produkt des realen geometrischen bzw. physischen Abstands I und dem Brechungsindex n des Mediums bzw. Fluids in dem Hohlraum 30. Der physische Abstand zwischen Ausgangselement 22 und Hornhaut bzw. Cornea 45 in Fig. 3 und Fig. 4 ist also identisch, während die optische Weglänge 71, 72 unterschiedlich groß ist.

In Fig. 3 und in Fig. 4 ist die Wand des Hohlraums 30 nicht dargestellt.

Eine erste Möglichkeit zur Bestimmung des Spiegels bzw. Füllstands der Flüssigkeit in dem Hohlraum 30 ist, eine Messung mittels optischer Kohärenztomografie (OCT) und/oder mittels Scheimpflug-Kamera zwischen dem Ausgangselement 22 des Flüssigkeits-Patienteninterfaces 20 (präziser: der in Fig. 3 bzw. Fig. 4 unteren Seite des Ausgangselements 22) und dem Auge 40 bzw. der Cornea 45 des Auges 40 (präziser: der in Fig. 3 bzw. Fig. 4 oberen Seite der Cornea 45 des Auges 40) durchzuführen. Hierbei wird die optische Weglänge 70, 72 (auch Abstand genannt) zwischen dem Ausgangselement 22 und dem Auge 40 bzw. der Cornea 45 bestimmt.

Die optische Weglänge 70 zwischen der Cornea 45 des Auges 40 bzw. des Auges 40 und dem Ausgangselement 22 des Flüssigkeits-Patienteninterfaces 20 ist die kürzeste optische Weglänge 70 zwischen der Cornea 45 des Auges 40 bzw. des Auges 40 und dem Ausgangselement 22 des Flüssigkeits-Patienteninterfaces 20. Anders ausgedrückt ist die optische Weglänge 70 zwischen dem Ausgangselement 22 und der Cornea 45 die optische Weglänge 70 zwischen dem Ausgangselement 22 und der Cornea 45 an der Stelle, an der die kürzeste physische Distanz zwischen dem Ausgangselement 22 und der Cornea 45 vorhanden ist.

Neben dieser optischen Weglänge 70 an der Stelle, an der die kürzeste physische Distanz zwischen dem Ausgangselement 22 und der Cornea 45 vorhanden ist, gibt es eine weitere optische Weglänge 71 zwischen dem Ausgangselement 22 und dem Kontaktelement 25. Neben diesen beiden optischen Weglängen 70, 71 gibt es eine weitere optische Weglänge 72, die an einer beliebigen Stelle zwischen dem Ausgangselement 22 und der Cornea 45 bestimmt wird.

Wenn der Hohlraum 30 mit Flüssigkeit (z.B. der BSS) im Wesentlichen vollständig gefüllt ist, gilt:

$$s_{BSS} = l * n_{BSS}$$

wobei

I der physische Abstand bzw. die physische Distanz zwischen dem Ausgangselement 22 und dem Auge 40 bzw. der Cornea 45 ist,
$n_{BSS}$ der Brechungsindex der Flüssigkeit (z.B. der BSS) ist, und
$s_{BSS}$ die optische Weglänge 70, 72 zwischen dem Ausgangselement 22 und dem Auge 40 bzw. der Cornea 45 ist, wenn der Hohlraum 30 mit Flüssigkeit (z.B. der BSS) gefüllt ist.

Wenn der Hohlraum 30 nicht mit Flüssigkeit sondern mit Luft gefüllt ist, gilt für die optische Weglänge 70, 72:

$$s_{Luft} = l * n_{Luft}$$

wobei

I der physische bzw. geometrische Abstand bzw. die physische Distanz zwischen dem Ausgangselement 22 und dem Auge 40 bzw. der Cornea 45 ist,
$n_{Luft}$ der Brechungsindex von Luft ist, und
$s_{Luft}$ die optische Weglänge 70, 72 zwischen dem Ausgangselement 22 und dem Auge 40 bzw. der Cornea 45 ist, wenn der Hohlraum 30 mit Luft gefüllt ist.

Da der Brechungsindex der BSS größer ist als der Brechungsindex von Luft, ist die optische Weglänge 70, 72 bei vollständiger bzw. teilweiser Füllung des Hohlraums 30 mit BSS größer als bei Vorhandensein von Luft in dem Hohlraum 30. Der Brechungsindex der BSS beträgt ca. 1,33 (nsss=1,33). Der Brechungsindex von Luft beträgt ca. 1,00.

Daher kann aus den gemessenen optischen Weglängen 70, 71 auf den Spiegel bzw. Füllstand der Flüssigkeit in dem Hohlraum 30 geschlossen werden bzw. darauf geschlossen werden, ob der Hohlraum 30 vollständig mit der Flüssigkeit (z.B. BSS) gefüllt ist oder nicht.

Wenn der Abstand bzw. die geometrische Länge bzw. der physische Abstand zwischen dem Ausgangselement 22 und dem Auge 40 beispielsweise 2 mm beträgt, ergibt sich (bei BSS als Flüssigkeit) ein Unterschied in der optischen Weglänge 70, 72 von ca. 660 μm:

$$s_{BSS} - s_{Luft} = 660 \ \mu m$$

Bei einer teilweisen Füllung des Hohlraums 30 mit einer Flüssigkeit, z.B. der BSS, verlängert sich die optische Weglänge 70, 72 (auch optischer Abstand genannt) gegenüber dem Vorhandensein von Luft in dem Hohlraum 30 nicht um die volle Distanz (d.h. um den Faktor 1,33 bei dem genannten Beispiel), sondern nur um einen entsprechenden Anteil:

$$s_{teilweise} = l_1 * n_{Luft} + l_2 * n_{BSS}$$

wobei

$n_{Luft}$ der Brechungsindex von Luft ist,
nsss der Brechungsindex der Flüssigkeit (z.B. der BSS) ist,
$s_{teilweise}$ die optische Weglänge 70, 72 zwischen dem Ausgangselement 22 und dem Auge 40 bzw. der Cornea 45 ist, wenn der Hohlraum 30 teilweise mit der Flüssigkeit (z.B. BSS) und teilweise mit Luft gefüllt ist,
$l_1$ die Höhe des Hohlraums 30 ist (die Höhe verläuft in Fig. 2a, Fig. 2b, Fig. 2c , Fig. 3 und Fig. 4 von oben nach unten), die mit Luft gefüllt ist,
$l_2$ die Höhe des Hohlraums 30 ist (die Höhe verläuft in Fig. 2a, Fig. 2b, Fig. 2c, Fig. 3 und Fig. 4 von oben nach unten), die mit der Flüssigkeit (z.B. BSS) gefüllt ist, und
$l=l_1+l_2$ die Gesamthöhe des Hohlraums zwischen dem Ausgangselement 22 und dem Auge 40 ist.

Gleiches gilt entsprechend für die Bestimmung/Berechnung der optischen Weglänge 71 zwischen dem Ausgangselement 22 und dem Kontaktelement 25.

Die optische Kohärenztomografie hat üblicherweise eine Auflösung von ca. 20 μm. Somit ist ein Abnehmen des Spiegels bzw. Füllstands der Flüssigkeit in dem Hohlraum 30 (gegenüber einer vollständigen Füllung des Hohlraums 30) rechtzeitig erkennbar. Der Spiegel bzw. Füllstand der Flüssigkeit lässt sich somit mit großer Präzision bestimmen. Folglich lässt sich präzise bestimmen, ob der Hohlraum 30 bzw. Zwischenraum vollständig mit der Flüssigkeit gefüllt ist bzw. inwieweit der Hohlraum 30 mit Flüssigkeit gefüllt ist.

Bevorzugt wird die optische Weglänge 70, 71, 72 am Apex bzw. am Scheitel bzw. auf der optischen Achse des Ausgangselements 22 bzw. der Ausgangslinse durchgeführt. Auf der optischen Achse des Ausgangselements 22 werden die optischen Strahlen (z.B. ein Laserstrahl des optischen Kohärenztomografiegeräts oder die optischen Messstrahlen) im Wesentlichen nicht abgelenkt.

Die Bestimmung der optischen Weglänge 70, 71, 72 zwischen dem Ausgangselement 22 und dem Auge 40 bzw. zwischen dem Ausgangselement 22 und dem Kontaktelement 25 wird einmal zu einem ersten Zeitpunkt durchgeführt, an dem der Hohlraum 30 des Flüssigkeits-Patienteninterface 20 sicher bzw. zuverlässig im Wesentlichen vollständig mit Flüssigkeit gefüllt ist. Dies kann z.B. unmittelbar nach dem Befüllen des Hohlraums 30 mit der Flüssigkeit sein. Die hierbei bestimmte optische Weglänge 70, 71, 72 wird als Referenzwert genommen.

Die Bestimmung bzw. Messung des aktuellen Spiegels/Füllstands der Flüssigkeit in dem Hohlraum 30 des Flüssigkeits-Patienteninterfaces 20 wird zu einem zweiten Zeitpunkt bzw. zweiten Zeitpunkten einmal oder mehrfach durchgeführt. Der erste Zeitpunkt liegt vor dem zweiten Zeitpunkt. Durch Vergleich des Referenzwerts mit dem bestimmten bzw. gemessenen Wert zum zweiten Zeitpunkt kann der Spiegel bzw. Füllstand der Flüssigkeit in dem Hohlraum 30 bestimmt werden. Der Vergleich kann z.B. durch Differenzbildung und/oder Verhältnisbildung der beiden Werte (Referenzwert und Messwert zum zweiten Zeitpunkt) durchgeführt werden. Somit kann bestimmt werden, inwieweit der Hohlraum 30 nicht (mehr) vollständig mit der Flüssigkeit gefüllt ist.

Bei der optischen Kohärenztomografie (Englisch: optical coherence tomography, OCT) wird Licht bzw. werden optische Messstrahlen geringer Kohärenzlänge aus dem Kurzpuls-Laser-Augenchirurgiegerät 10 in das Flüssigkeits-Patienteninterface 20 gesandt bzw. abgestrahlt. Die Rückstreuung und/oder Reflexion der Grenzfläche zwischen dem Ausgangselement 22 und dem Hohlraum 30 und/oder zwischen vorhandener Luft und der Flüssigkeit und/oder zwischen dem Hohlraum 30 und dem Auge 40 bzw. der Cornea 45 des Auges 40 (d.h. Rückstreuung und/oder Reflexion aus dem Flüssigkeits-Patienteninterface 20) werden in dem Kurzpuls-Laser-Augenchirurgiegerät 10 erfasst. Somit bedarf es keiner Erfassungsvorrichtung, Messvorrichtung oder ähnlichem in dem Flüssigkeits-Patienteninterface 20, wodurch das Flüssigkeits-Patienteninterface 20 (das bei jedem Patienten gewechselt werden muss, um Infektionen zu verhindern) technisch einfach ausgebildet sein kann und kostengünstig herstellbar ist. Das optische Kohärenztomografiegerät kann die Strahlerzeugungsvorrichtung 85 zum Aussenden von optischen Messstrahlen aus dem Kurzpuls-Laser-Augenchirurgiegerät in das Flüssigkeits-Patienteninterface, die Erfassungsvorrichtung 86 und die Bestimmungsvorrichtung umfassen. Anders ausgedrückt können die Strahlerzeugungsvorrichtung 85, die Erfassungsvorrichtung 86 und die Bestimmungsvorrichtung Teile des optischen Kohärenztomografiegeräts sein.

Alternativ oder zusätzlich zur optischen Kohärenztomografie kann die optische Weglänge 70, 71 zwischen dem Ausgangselement 22 des Flüssigkeits-Patienteninterfaces 20 und dem Auge 40 bzw. der Cornea 45 des Auges 40 mittels einer Scheimpflug-Kamera bzw. einer Messung mit einer Scheimpflug-Kamera bestimmt werden.

Bei der Messung mittels einer Scheimpflug-Kamera bzw. -Kameras wird ein Bild oder eine Vielzahl von Bildern hergestellt. Die Scheimpflug-Kamera kann derart ausgebildet sein, dass die Scheimpflug-Kamera um das Auge 40 rotiert. Auf Grundlage der Scheimpflugschen Regel bzw. der Scheimpflug-Bedingung wird die (kürzeste) optische Weglänge (auch Abstand genannt) zwischen dem Ausgangselement 22 des Flüssigkeits-Patienteninterfaces 20 und der Cornea 45 des Auges 40 (oder dem Kontaktelement 25 Flüssigkeits-Patienteninterfaces 20) bestimmt. Die Scheimpflug-Kamera ist Teil des Kurzpuls-Laser-Augenchirurgiegeräts 10. Die Scheimpflug-Kamera ist in diesem Fall die Strahlerzeugungsvorrichtung 85 zum Aussenden von optischen Messstrahlen aus dem Kurzpuls-Laser-Augenchirurgiegerät in das Flüssigkeits-Patienteninterface und zugleich die Erfassungsvorrichtung 86. Auch die Bestimmungsvorrichtung kann Teil der Scheimpflug-Kamera sein.

Wenn festgestellt wird, dass der Hohlraum 30 nicht im Wesentlichen vollständig mit der Flüssigkeit (z.B. BSS) gefüllt ist oder der Füllstand der Flüssigkeit unterhalb der kritischen Grenze bzw. einem kritischen Grenzwert liegt, wird ein Warnsignal erzeugt. Das Warnsignal kann ein digitales oder analoges Warnsignal sein. Durch das Warnsignal kann ein optisches und/oder akustisches Signal erzeugt werden, um den Bediener des Kurzpuls-Laser-Augenchirurgiesystems 5 darauf hinzuweisen, dass der Hohlraum 30 nicht (mehr) im Wesentlichen vollständig mit der Flüssigkeit gefüllt ist. Auch eine Anzeige des Füllstands mittels einer analogen und/oder digitalen Anzeige ist möglich. Das Warnsignal kann auch dazu dienen, den Laserstrahl des Kurzpuls-Laser-Augenchirurgiegeräts 10 zur Behandlung des Auges 40 sofort bzw. unmittelbar abzuschalten und/oder ein Einschalten des Laserstrahls zu verhindern. Wenn der Hohlraum 30 nicht vollständig mit der Flüssigkeit gefüllt ist, kann der Laserstrahl des Kurzpuls-Laser-Augenchirurgiegeräts 10 nicht mit der gewünschten bzw. geplanten Präzision in dem Auge 40 geführt werden und damit zu ungewollten Beschädigungen im Auge und/oder von Komponenten des Kurzpuls-Laser-Augenchirurgiesystems 5 führen. Eine erfolgreiche Behandlung des Auges 40 mit dem Laserstrahl des Kurzpuls-Laser-Augenchirurgiegeräts 10 kann in diesem Fall nicht sichergestellt werden, so dass eine Behandlung abgebrochen wird bzw. der Start der Behandlung verhindert wird. Das Warnsignal kann den Nutzer darauf hinweisen, dass die Flüssigkeit nachgefüllt werden muss, um im Therapie-Prozess voranschreiten zu können. Erst wenn der Hohlraum 30 wieder vollständig mit Flüssigkeit gefüllt ist, wird das erneute Starten des Laserstrahls ermöglicht und/oder durchgeführt.

Ein Ausfließen von Flüssigkeit aus dem Hohlraum 30 ist z.B. durch eine Undichtigkeit des Kontaktelements 25 und/oder dem Entfernen des Auges 40 von dem Kontaktelement 25 bzw. dem Flüssigkeits-Patienteninterfaces 20 möglich.

Eine im Wesentlichen vollständige Füllung des Hohlraums 30 mit der Flüssigkeit kann insbesondere bedeuten, dass der Hohlraum 30 (vom Auge 40) bis zu der Marke 33 gefüllt ist. Der Bereich bzw. Teil des Hohlraums 30, der (in Fig. 2a bzw. Fig. 2b) oberhalb der Marke 33 liegt, ist für die Bestimmung, ob der Hohlraum 30 vollständig mit Flüssigkeit gefüllt ist oder nicht unbeachtlich. D.h., auch wenn in diesem Bereich keine Flüssigkeit vorhanden ist und der Hohlraum 30 (von dem Auge 40) bis zur Marke 33 mit Flüssigkeit gefüllt ist, ist bzw. gilt der Hohlraum 30 im Wesentlichen vollständig mit Flüssigkeit gefüllt. Der Laserstrahl zur Behandlung des Auges 40 dringt in der Regel in diese Bereiche nicht ein, so dass das Nichtvorhandensein von Flüssigkeit in diesem Bereich des Hohlraums 30 keine negativen Auswirkungen auf die Sicherheit und Zuverlässigkeit der Behandlung des Auges 40 mit dem Laserstrahl hat.

Der bestimmte erste Messwert wird mit dem Referenzwert verglichen, um den Spiegel bzw. Füllstand der Flüssigkeit in dem Hohlraum 30 zu bestimmen.

Alternativ oder zusätzlich zu der Bestimmung der optischen Weglänge 70, 71, 72 zwischen dem Ausgangselement 22 des Flüssigkeits-Patienteninterfaces 20 und dem Auge 40 zur Bestimmung des Spiegels bzw. Füllstands der Flüssigkeit in dem Hohlraum 30 kann eine optische Weglänge 71 zwischen dem Ausgangselement 22 des Flüssigkeits-Patienteninterfaces 20 und einem Kontaktelement 25 des Flüssigkeits-Patienteninterfaces 20 zum unmittelbaren Kontaktieren des Auges 40 bestimmt werden. Das Flüssigkeits-Patienteninterface 20 weist auf der dem Ausgangselement 22 abgewandten Seite des Hohlraums 30 ein Kontaktelement 25 auf. Das Kontaktelement 25 ist im Querschnitt senkrecht zur Richtung 15 des Laserstrahls üblicherweise kreis- oder ellipsenförmig ausgebildet. Das Kontaktelement 25 dient zum unmittelbaren Kontaktieren des Auges 40 bzw. der Cornea 45 des Auges 40 und Fixieren des Auges 40. Das Kontaktelement 25 ist üblicherweise aus einem weichen Material bzw. flexiblen Material hergestellt. Das Material kann z.B. einen weichen Kunststoff umfassen oder aus diesem bestehen. Das Material kann insbesondere Silikon umfassen. Das Kontaktelement 25 dient als flüssigkeitsdichte (oder gegebenenfalls luftdichte) Abdichtung des Zwischenraums zwischen der seitlichen Wand 35 des Flüssigkeits-Patienteninterfaces 20 und dem Auge 40 bzw. der Cornea 45.

Wenn sich das Auge 40 in unmittelbarem Kontakt mit dem Kontaktelement 25 befindet (wie in Fig. 2a gezeigt), ist der Hohlraum 30 vollständig abgeschlossen bzw. verschlossen. Das Kontaktelement 25 sorgt dafür, dass das Auge 40 relativ zum Flüssigkeits-Interface und folglich relativ zum Kurzpuls-Laser-Augenchirurgiegerät 10 nicht (bewusst oder unbewusst) bewegt bzw. verdreht werden kann. Hierdurch ist die Sicherheit bei der gezielten Behandlung des Auges 40 mit dem Laserstrahl aus dem Kurzpuls-Laser-Augenchirurgiegerät 10 sichergestellt. Der Laserstrahl kann präzise in Bezug auf das Auge 40 positioniert bzw. auf dem Auge 40 geführt werden.

Der physische bzw. geometrische Abstand zwischen dem Ausgangselement 22 des Flüssigkeit-Patienteninterfaces und dem Kontaktelement 25 ist stets gleich groß. Auch bei baugleichen unterschiedlichen Flüssigkeits-Patienteninterfaces 20 und bei unterschiedlichen Patientenaugen 40 ist (innerhalb der Herstellungstoleranzen) diese physikalische Distanz bzw. dieser physische Abstand stets gleich groß. Da der Brechungsindex der Flüssigkeit (z.B. BSS) bekannt ist, kann die optische Weglänge 71 zwischen dem Ausgangselement 22 des Flüssigkeits-Patienteninterfaces 20 und dem Kontaktelement 25 bei vollständiger Füllung des Hohlraums 30 mit der Flüssigkeit aus dem geometrischen Abstand bestimmt bzw. berechnet werden (diese optische Weglänge 71 ist der fest vorgegebene Referenzwert). Somit muss zur Bestimmung des Spiegels der Flüssigkeit in dem Hohlraum 30 hierbei keine zweite Messung zur Bestimmung eines Referenzwerts durchgeführt werden. Alternativ wird diese Messung einmalig für eine Bauart des Flüssigkeits-Patienteninterfaces 20 durchgeführt und gespeichert, so dass der gemessene Wert als Referenzwert für alle baugleichen Flüssigkeits-Patienteninterfaces 20 verwendet werden kann.

Eine in-situ Messung bzw. Messung/Bestimmung der optischen Weglänge 71 bei vollständiger Füllung des Hohlraums 30 mit der Flüssigkeit und an dem Flüssigkeits-Patienteninterface 20 angeordnetem Auge 40, das den Hohlraum 30 verschließt, ist somit nicht notwendig (sie kann aber dennoch ausgeführt werden). Nur eine Messung/Bestimmung zum zweiten Zeitpunkt, an dem bzw. wenn der Spiegel bzw. Füllstand der Flüssigkeit in dem Hohlraum 30 bestimmt werden soll, muss durchgeführt werden. In diesem Fall ist der Referenzwert ein fest vorgegebener Referenzwert.

Auch bei Verwendung eines fest vorgegeben Referenzwerts kann eine teilweise Füllung des Hohlraums 30 mit der Flüssigkeit festgestellt werden. Der Referenzwert selbst kann aus mehreren einzelnen Referenzwertelementen bestehen.

Die optische Weglänge 71 kann hierbei durch optische Kohärenztomografie und/oder durch Messung mittels Scheimpflug-Kamera bestimmt werden.

Eine weitere Möglichkeit zur Bestimmung des Spiegels der Flüssigkeit in dem Hohlraum 30 bzw. der Füllstandmessung ist die Bestimmung einer Intensität der aus dem Flüssigkeits-Patienteninterface 20 und/oder von dem Auge 40 reflektierten und/oder rückgestreuten optischen Messstrahlen, d.h. der Reflexion und/oder Rückstreuung der optischen Messstrahlen. Die optischen Messstrahlen können Strahlen einer optischen Kohärenztomografie bzw. eines optischen Kohärenztomografiegeräts sein. Die Intensität (auch "Amplitude" genannt) gibt an wieviel Licht bzw. optische Messstrahlen oder welcher Anteil des ausgesandten Lichts zurückgeworfen wird/werden. Hierbei wird z.B. lediglich die Intensität von Reflexion und/oder Rückstreuung der optischen Messstrahlen der optischen Kohärenztomografie bestimmt. Es wird üblicherweise neben der Bestimmung der Intensität keine weitere Auswertung/Berechnung der Reflexion und/oder Rückstreuung durchgeführt. Wenn die Flüssigkeit in dem Hohlraum 30 den Brechungsindexunterschied zwischen dem Ausgangselement 22 und dem Auge 40 nivelliert, ist die Reflexion/Rückstreuung an der Grenzfläche zwischen dem Ausgangselement 22 und dem Hohlraum 30 geringer, als wenn der Hohlraum 30 nicht im Wesentlichen vollständig mit der Flüssigkeit gefüllt ist. Gleiches gilt für die Grenzflächen zwischen dem Hohlraum 30 und dem Auge 40 bzw. der Cornea 45 des Auges 40. Somit wird eine geringe Intensität der Reflexion und/oder Rückstreuung der optischen Messstrahlen gemessen, wenn der Hohlraum 30 vollständig mit der Flüssigkeit gefüllt ist. Ein Ansteigen der Intensität bzw. ein großer/größerer Wert als zuvor der Intensität zeigt an, dass der Hohlraum 30 nicht mehr vollständig mit Flüssigkeit gefüllt ist.

Somit wird hierbei typischerweise keine optische Weglänge 70, 71, 72 mittels der optischen Kohärenztomografie bestimmt, sondern nur die Intensität bzw. Stärke der Reflexion und/oder Rückstreuung der optischen Messstrahlen bzw. der reflektierten und/oder rückgestreuten optischen Messstrahlen bestimmt. Die Intensität ist in diesem Fall der erste Messwert. Im vollständig gefüllten Zustand des Hohlraums 30 mit der Flüssigkeit, ist die Intensität (im Bereich der Pupille/Linse 41 des Auges 40) sehr klein oder nahe Null.

Auch möglich ist, dass mehrere erste Messwerte erfasst werden. Z.B. können die mehreren ersten Messwerte die optischen Weglängen 70, 71, 72 zwischen dem Ausgangselement 22 des Flüssigkeits-Patenteninterfaces 20 und dem Auge 40 oder dem Kontaktelement 25 an unterschiedlichen Stellen des Hohlraums 30 sein. Die unterschiedlichen Stellen können in Fig. 2a von links nach rechts an unterschiedlichen Positionen sein.

Die Rückstreuung und/oder Reflexion der optischen Messstrahlen bzw. die rückgestreuten und/oder reflektierten optischen Messstrahlen werden in dem Kurzpuls-Laser-Augenchirurgiegerät 10 mittels einer Erfassungsvorrichtung 86 erfasst. Die Erfassungsvorrichtung 86 kann z.B. ein Lichtsensor sein oder umfassen.

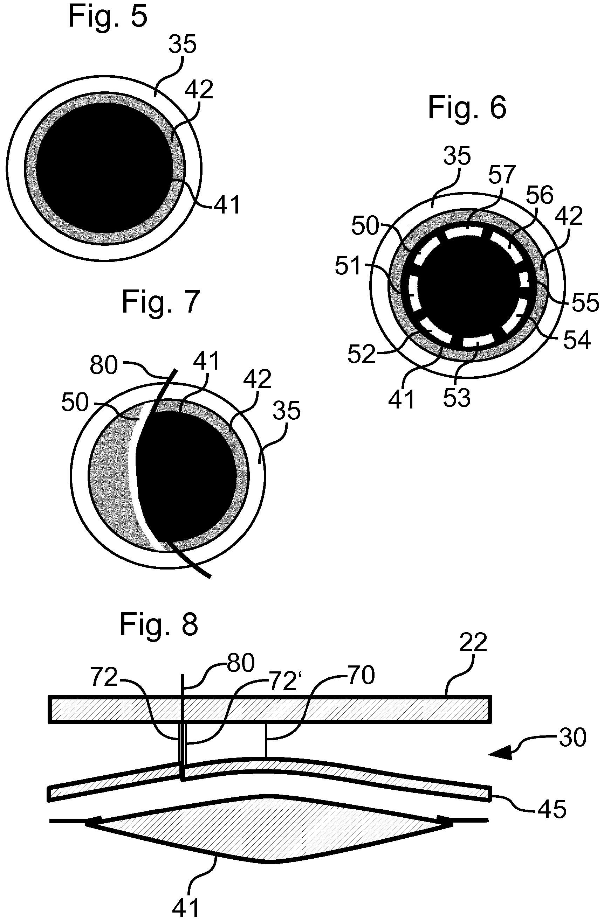

Fig. 5 zeigt eine schematische Aufsicht auf das Auge 40 durch das Flüssigkeits-Patenteninterface 20 bei im Wesentlichen vollständiger Füllung des Hohlraums 30 mit der BSS. Fig. 6 zeigt eine schematische Aufsicht auf das Auge 40 durch das Flüssigkeits-Patenteninterface 20 bei nicht im Wesentlichen vollständiger Füllung des Hohlraums 30 mit der BSS.

Fig. 5 und Fig. 6 zeigen jeweils eine sogenannte Draufsicht bzw. ein Draufsichtbild.

Eine weitere Möglichkeit zum Bestimmen des Spiegels bzw. Füllstands der Flüssigkeit in dem Hohlraum 30 ist die Bildanalyse eines oder mehrerer aufgenommener Bilder. Hierbei wird sichtbares Licht und/oder Infrarot-Licht aus dem Kurzpuls-Laser-Augenchirurgiegerät 10 in das Flüssigkeits-Patenteninterface 20 ausgesandt, um Reflexion und/oder Rückstreuung des sichtbaren Lichts und/oder des Infrarot-Lichts in dem Kurzpuls-Laser-Augenchirurgiegerät 10 zu detektieren.

Falls der Hohlraum 30 im Wesentlichen vollständig mit der Flüssigkeit gefüllt ist, treten aufgrund der Brechungsindex-Anpassung im Wesentlich keine Reflexion und/oder Rückstreuung in dem Flüssigkeits-Patenteninterface 20 im Bereichs des Auges 40 auf. In Fig. 5 und in Fig. 6 stellt der hell dargestellte Bereich innerhalb des äußersten Kreises die seitliche Wand 35 des Flüssigkeits-Patenteninterfaces 20 dar. Im Bereich des Auges 40, der grau (zweitäußerster Kreis; Randbereich 42/Iris des Auges 40) und schwarz (Pupille/Linse 41 des Auges 40) dargestellt ist, gibt es bei im Wesentlichen vollständiger Füllung des Hohlraums 30 mit der Flüssigkeit nur eine sehr geringe Reflexion und/oder Rückstreuung (grauer Bereich) bzw. keine Reflexion und/oder Rückstreuung (schwarzer Bereich). Es treten im Bereich des Auges 40 bzw. der Pupille/Linse 41 keine sogenannten Beleuchtungsreflexe/Reflexionen/Rückstreuungen auf, wenn der Hohlraum 30 zwischen Ausgangselement 22 und Auge 40 vollständig gefüllt ist, wie in Fig. 5 gezeigt.

Falls der Hohlraum 30 nicht oder nicht im Wesentlichen vollständig mit der Flüssigkeit gefüllt ist, wird zumindest ein Teil des aus dem Kurzpuls-Laser-Augenchirurgiegerät 10 ausgesandten Lichts (bzw. der optischen Messstrahlen) reflektiert und/oder rückgestreut und aus dem Flüssigkeits-Patenteninterface 20 in das Kurzpuls-Laser-Augenchirurgiegerät 10 zurückgeworfen. In dem Kurzpuls-Laser-Augenchirurgiegerät 10 werden die rückgestreuten und/oder reflektierten optischen Messstrahlen bzw. die Rückstreuung und/oder Reflexion der von dem Kurzpuls-Laser-Augenchirurgiegerät 10 in das Flüssigkeits-Patenteninterface 20 ausgesandten optischen Messstrahlen von einer Erfassungsvorrichtung 86 (Kamera) erfasst.

Es wird hierbei (zu dem zweiten Zeitpunkt) ein Bild mittels der visuellen Kamera oder der IR-Kamera aufgenommen und das Bild in einer Bildanalysevorrichtung (z.B. einem Computer mit entsprechender Analyse-Software zum Erkennen der Reflexion und/oder Rückstreuung bzw. des reflektierten und/oder rückgestreuten Lichts) analysiert. Die optischen Messstrahlen können in diesem Fall (inkohärentes) sichtbares Licht oder Infrarotlicht sein.

Wie in Fig. 6 deutlich zu erkennen, können im Bereich des Auges 40 bzw. der Pupille/Linse 41 des Auges 40 Reflexion und/oder Rückstreuungen z.B. aufgrund einer Beleuchtungsvorrichtung des Kurzpuls-Laser-Augenchirurgiegeräts auftreten, wenn der Hohlraum 30 zwischen Ausgangselement 22 und Auge 40 nicht oder nicht vollständig gefüllt ist. Dies ist in Fig. 6 beispielhaft in Form kranzartiger weißer Bereiche unterschiedlicher Größe im Falle einer kranzförmigen, strukturierten Ringbeleuchtung dargestellt. Die Reflexion entsteht durch den großen Brechungsindexunterschied zwischen Hornhaut bzw. Cornea 45 und der Luft in Hohlraum 30 und tritt im gesamten Bereich des Flüssigkeits-Patenteninterfaces 20 auf. Die Größe, Form und Strukturierung der Beleuchtung ist für die hier diskutierte Lösung nicht relevant, da jede beliebige Beleuchtung bei luftgefülltem Hohlraum 30 Reflexionen auf der Hornhaut bzw. Cornea 45 zeigt.

Das in Fig. 6 dargestellte Beispiel einer unvollständigen Befüllung des Hohlraums 30 mit Flüssigkeit umfasst eine beispielhafte Ringbeleuchtung mit einem relativ großen Durchmesser (in etwa so groß wie die Pupille). Das bedeutet für den in Fig. 6 dargestellten Fall, dass sich im Hohlraum 30 fast kein BSS oder gar kein BSS befindet. Sobald man BSS in den Hohlraum 30 füllen würde, so dass das BSS bis hinauf zur Marke 33 die reflektierenden Hornhautbereiche überdeckt, würden die Reflexionen der Ringbeleuchtung aufgrund der Brechungsindexanpassung verschwinden und man erhielte bei im Wesentlichen vollständiger Füllung des Hohlraums mit Flüssigkeit, d.h. bis zur Marke 33, eine Draufsicht wie in Fig. 5.

Durch die Bildanalyse kann das Vorhandensein eines Flüssigkeitsspiegels unterhalb des Ausgangselements 22 bzw. der Füllstand der Flüssigkeit in dem Hohlraum 30 qualitativ auf Basis von Reflexionen auf der Hornhaut bzw. Cornea 45 bestimmt werden. Z.B. können bei der Bildanalyse sehr helle Stellen des Bilds (sogenannte Beleuchtungsreflexe 50-57) erkannt werden, die durch Reflexion und/oder Rückstreuung der optischen Messstrahlen entstehen. Das Vorhandensein, die Helligkeit und/oder die Anzahl der Beleuchtungsreflexe können hierbei bestimmt werden. Hieraus wird bzw. werden der erste Messwert oder die ersten Messwerte bestimmt.

Vorstellbar ist auch, dass der Referenzwert das Draufsichtbild ist, das zu einem ersten Zeitpunkt erfasst wird, wenn der Hohlraum 30 (sicher) vollständig mit der Flüssigkeit gefüllt ist. Diese Draufsicht ist in Fig. 5 dargestellt. Der mindestens eine erste Messwert ist dann das Bild in Draufsicht, das zu einem zweiten Zeitpunkt, zur Bestimmung des Spiegels bzw. Füllstands des Hohlraums 30 angefertigt wird oder der hieraus berechnete Wert. Solch eine Art Bild ist exemplarisch in Fig. 6 dargestellt.

Fig. 7 zeigt eine schematische Aufsicht auf das Auge 40 durch das Flüssigkeits-Patienteninterface 20 bei teilweiser Füllung des Hohlraums 30 mit der BSS. Fig. 8 zeigt eine schematische Querschnittsansicht des Hohlraums 30 bei teilweiser Füllung des Hohlraums 30 mit der BSS. Fig. 7 zeigt ein Draufsicht-Bild. Fig. 8 zeigt ein Bild der optischen Kohärenztomografie oder alternativ das Bild einer Scheimpflug-Kamera.

In Fig. 7 bzw. Fig. 8 ist der linke Teil bzw. linke Bereich (links von der Flüssigkeitsgrenze 80) des Hohlraums 30 nicht mehr mit Flüssigkeit gefüllt, d.h. dieser Bereich ist trocken. Hier ist in Fig. 7 deutlich zu sehen, dass im Bereich der Pupille/Linse 41 Reflexion und/oder Rückstreuung in diesem linken Bereich auftritt. Der rechte Teil bzw. rechte Bereich (rechts von der Flüssigkeitsgrenze 80) ist teilweise oder vollständig mit der Flüssigkeit (z.B. BSS) gefüllt. In diesem rechten Bereich des Hohlraums 30 tritt weniger bis keine Reflexion und/oder Rückstreuung auf.

Der Unterschied zwischen diesen beiden Bereichen (links von der Flüssigkeitsgrenze 80 und rechts von der Flüssigkeitsgrenze 80) ist in Fig. 8 deutlich zu erkennen. Die optische Weglänge 72, 72' weist einen Knick bzw. Sprung beim Übergang von dem mit BSS gefüllten Bereich (rechter Bereich, rechts der Flüssigkeitsgrenze 80) zu dem trockenen Bereich (linker Bereich, links von der Flüssigkeitsgrenze 80) auf. Im trockenen Bereich ist die optische Weglänge 72, 72' zwischen dem Ausgangselement 22 und dem Auge 40 bzw. der Cornea 45 des Auges 40 geringer als im feuchten/mit BSS teilweise gefüllten Bereich.

Bei der Verwendung der Kamera mit Licht im sichtbaren Bereich (visuelle Kamera) und/oder der Infrarotkamera sollte sich die Cornea 45 des Auges 40 im Scharfbereich des Ausgangselements 22 bzw. der Ausgangslinse des Flüssigkeits-Patienteninterfaces 20 befinden. Das Beleuchtungsspektrum, d.h. das Spektrum der von dem Kurzpuls-Laser-Augenchirurgiegerät 10 in das Flüssigkeits-Patienteninterface 20 ausgesandten optischen Messstrahlen, ist auf die Kamera angepasst, so dass Reflexion und/oder Rückstreuung der optischen Messstrahlen in dem Kurzpuls-Laser-Augenchirurgiegerät 10 technisch einfach erfasst werden kann.

Die optische Kohärenztomografie kann entweder der Art Time-Domain, Frequency-Domain oder Swept-Source sein. Die Wellenlänge des als Messstrahlen genutzten Laserstrahls der optischen Kohärenztomografie kann variabel bzw. anpassbar sein. Gleiches gilt für die Intensität des Laserstrahls.

Bei der Verwendung der Scheimpflug-Kamera können die Art und das Spektrum der Lichtquelle variabel angepasst werden. Gleiches gilt für die Art und das Detektionsspektrum des Kamerasystems für die Scheimpflug-Kamera.

Bei der Verwendung einer Draufsicht (Lichtkamera und/oder Infrarotkamera) können die Messstrahlen entweder koaxial zu der Richtung der Draufsicht-Kamera oder seitlich eingekoppelt sein. Wesentlich ist, dass die Beleuchtung bzw. die Messstrahlen zu Reflexionen und/oder Rückstreuungen an der Cornea 45 und/oder am Übergang von dem Ausgangselement 20 und dem Hohlraum 30 und/oder an der Grenze Luft/Flüssigkeit führen, wenn der Hohlraum 30 des Flüssigkeits-Patienteninterfaces 20 nicht vollständig oder unterhalb eines Grenzwerts mit der Flüssigkeit bzw. BSS gefüllt ist.

Reflexion und/oder Rückstreuung kann bei den genannten Arten der Bestimmung des Spiegels der Flüssigkeit in dem Hohlraum 30 des Flüssigkeits-Patienteninterfaces 20 auch jeweils dann auftreten, wenn der Hohlraum 30 zwar mit einer Flüssigkeit gefüllt ist, diese Flüssigkeit jedoch einen von dem Brechungsindex des Auges 40 bzw. der Ausgangslinse (stark) unterschiedlichen Brechungsindex aufweist. Dies bedeutet, dass die Brechungsindizes der Ausgangslinse, der Flüssigkeit und des Auges 40 bzw. der Cornea 45 nicht gegeneinander nivelliert bzw. sehr ähnlich sind. Auch in diesem Fall wird, da Reflexion und/oder Rückstreuung auftritt, ein Warnsignal erzeugt und der Betrieb des Laserstrahls des Kurzpuls-Laser-Augenchirurgiegeräts 10 zur Behandlung des Auges 40 gestoppt bzw. verhindert.

Die Rückstreuung entspricht der diffusen Reflexion bzw. ist die diffuse Reflexion. Die Reflexion entspricht der spiegelnden Reflexion bzw. ist die spiegelnde Reflexion. Rückstreuung und Reflexion zusammen sind die von dem Flüssigkeits-Patienteninterface, der Flüssigkeit und/oder dem Auge 40 in das Kurzpuls-Laser-Augenchirurgiegerät zurückgeworfenen optischen Messstrahlen bzw. das entsprechende Licht.

Das Kohärenztomografiegerät zur optischen Kohärenztomografie und/oder die Scheimpflug-Kamera (bzw. die Strahlerzeugungsvorrichtung 85 und/oder die Erfassungsvorrichtung 86) können zudem zur Bestimmung der Position und/oder Form des Auges 40 und seiner Strukturen verwendet werden. Die Position und/oder Form des Auges 40 sind für die präzise und sichere Behandlung des Auges 40 mit dem Laserstrahl des Kurzpuls-Laser-Augenchirurgiegeräts 10 wichtig bzw. relevant.

Auch Luftblasen innerhalb der Flüssigkeit in dem Hohlraum 30 sind durch das Kurzpuls-Laser-Augenchirurgiesystem 5 bzw. durch das Verfahren feststellbar. Da diese die Behandlung des Auges 40 mit dem Laserstrahl negativ beeinträchtigen können, kann auch beim Feststellen von Luftblasen in der Flüssigkeit der Laserstrahl gestoppt bzw. das Einschalten des Laserstrahls verhindert.

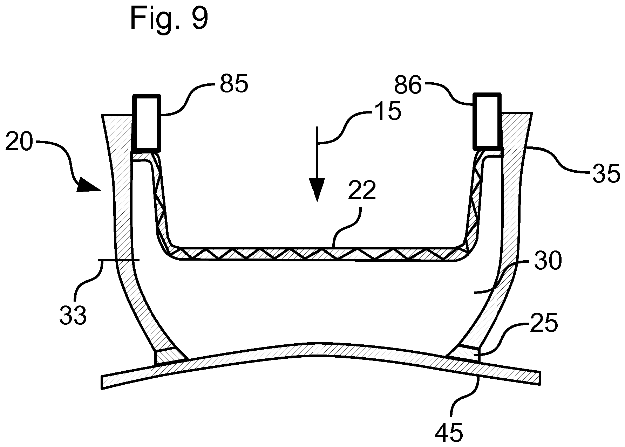

Fig. 9 zeigt eine detaillierte schematische Querschnittsansicht einer dritten Ausführungsform des Flüssigkeits-Patienteninterfaces 20 sowie eines Teils des Kurzpuls-Laser-Augenchirurgiegeräts 10, wobei der Hohlraum mit Luft gefüllt ist. Hierbei wird zum Bestimmen des Füllstands der Flüssigkeit in dem Hohlraum 30 eine Transmission der optischen Messstrahlen durch das Ausgangselement 22 als erster Messwert gemessen. Hierfür weist das Kurzpuls-Laser-Augenchirurgiegerät 10 eine Strahlerzeugungsvorrichtung 85 zum Aussenden der optischen Messstrahlen in das Ausgangselement 22 auf und eine Erfassungsvorrichtung 86 zum Erfassen der durch das Ausgangselement 22 transmittierten optischen Messstrahlen auf. Die Strahlerzeugungsvorrichtung 85 zum Aussenden der optischen Messstrahlen liegt direkt bzw. unmittelbar auf einer dem Hohlraum 30 abgewandten Seite des Ausgangselements 22 auf. Die Strahlerzeugungsvorrichtung 85 befindet sich an oder in der Nähe der seitlichen Wand 35 des Flüssigkeits-Patienteninterfaces 20. Die Strahlerzeugungsvorrichtung 85 sendet optische Messstrahlen in das Ausgangselement 22 entlang der Erstreckungsrichtung des Ausgangselements 22.

Die Erstreckungsrichtung des Ausgangselements 22 verläuft unterhalb der Strahlerzeugungsvorrichtung 85 nach unten bzw. ungefähr in Richtung des Laserstrahls 15, verläuft anschließend (in der Mitte) horizontal bzw. senkrecht zur Richtung des Laserstrahls 15 und schließlich wieder nach oben bzw. ungefähr entgegengesetzt zur Richtung des Laserstrahls 15. Durch interne Totalreflexion werden die optischen Messstrahlen mehrfach reflektiert und setzen sich entlang der Erstreckungsrichtung des Ausgangselements 22 in dem Ausgangselement 22 fort (wenn sich Luft in dem Hohlraum 30 bzw. an der den Hohlraum 30 zugewandten Seite des Ausgangselements 22 befindet). Die Strahlerzeugungsvorrichtung 85 zum Aussenden der optischen Messstrahlen kann z.B. eine Leuchtdiode (LED) und/oder ein Laser umfassen bzw. sein.

Die Erfassungsvorrichtung 86 ist ebenfalls unmittelbar bzw. direkt auf einer dem Hohlraum 30 abgewandten Seite des Ausgangselements 22 angeordnet. Die Erfassungsvorrichtung 86 ist an der seitlichen Wand 35 des Hohlraums 30 /Flüssigkeits-Patienteninterface 20 bzw. in der Nähe hiervon angeordnet. Die Erfassungsvorrichtung 86 ist auf einer Seite gegenüber der Strahlerzeugungsvorrichtung 85 zum Aussenden der optischen Messstrahlen angeordnet. Die Erfassungsvorrichtung 86 erfasst die durch das Ausgangselement 22 entlang der Erstreckungsrichtung des Ausgangselements 22 transmittierten optischen Messstrahlen.

Wenn sich keine Flüssigkeit in dem Hohlraum 30 befindet, werden im Wesentlichen die gesamten optischen Messstrahlen bzw. das gesamte Licht, das von der Strahlerzeugungsvorrichtung 85 bzw. LED ausgesandt wird, durch das Ausgangselement 22 geleitet (transmittiert) und gelangt zu der Erfassungsvorrichtung 86. Abgesehen von einer im Wesentlichen vernachlässigbaren Absorption in dem Ausgangselement 22 wird das gesamte Licht entlang des Ausgangselements 22 geleitet (durch interne Totalreflexion). Ein Intensitätssignal bzw. die Intensität des Transmissionssignals der optischen Messstrahlen, das bzw. die von der Erfassungsvorrichtung 86 erfasst wird, entspricht somit im Wesentlichen der Intensität des von der Strahlerzeugungsvorrichtung 85 zum Aussenden der optischen Messstrahlen ausgesandten Lichts.

Wenn der Hohlraum 30 im Wesentlichen vollständig mit Flüssigkeit gefüllt ist, so erfolgt an der dem Auge zugewandten Seite des Ausgangselements keine Totalreflexion mehr, da nun eine Grenzfläche zwischen dem Ausgangselement und der Flüssigkeit anstatt vorher zwischen dem Ausgangselement und Luft besteht. Deshalb gelangt ein beträchtlicher Teil der Intensität der ausgesandten optischen Messstrahlen bzw. des Lichts in dem Flüssigkeits-Patienteninterface 20 aus dem Ausgangselement 22 heraus und wird somit nicht in die Erfassungsvorrichtung 86 geleitet. Auch vorstellbar ist, dass die gesamte Intensität der ausgesandten optischen Messstrahlen das Ausgangselement 22 verlässt.

Durch Streuverluste gelangt also ein Teil der optischen Messstrahlen oder die gesamten optischen Messstrahlen aus dem Ausgangselement 22 heraus, bevor diese das Ausgangselement 22 entlang seiner Erstreckungsvorrichtung vollständig durchlaufen bzw. transmittiert haben. Die Streuverluste der Intensität des ausgesandten Lichts treten an den Stellen auf, an denen die Flüssigkeit das Ausgangselement 22 auf der Seite des Hohlraums 30 kontaktiert bzw. benetzt. An diesen Stellen ist der Unterschied in den Brechungsindizes zwischen dem Ausgangselement 22 und dem Medium in dem Hohlraum 30, nämlich hier der Flüssigkeit, derart gering, dass die Bedingung für interne Totalreflexion nicht mehr erfüllt ist. Daher tritt ein Teil der Intensität der optischen Messstrahlen aus dem Ausgangselement 22 (in die Flüssigkeit) heraus und geht damit für eine Erfassung in der Erfassungsvorrichtung 86 verloren.

Bei einer im Wesentlichen vollständigen Füllung des Hohlraums 30 mit Flüssigkeit bzw. bei einer Füllung des Hohlraums 30 von der Cornea 45 bis zu der Marke 33 sind die Streuverluste aufgrund des Austretens von optischen Messstrahlen aus dem Ausgangselement 22 (da im Wesentlichen keine interne Totalreflexion mehr auftritt) derart hoch, dass nur eine sehr geringe Intensität von optischen Messstrahlen oder gar keine optischen Messstrahlen mehr bei der Erfassungsvorrichtung 86 erfasst bzw. gemessen werden. Wenn der Hohlraum 30 von der Cornea 45 (d.h. unten) bis zu der Marke 33 mit Flüssigkeit gefüllt ist, ist der gesamte in Fig. 9 horizontal verlaufende Bereich des Ausgangselements 22 mit Flüssigkeit bedeckt bzw. benetzt.

Das Intensitätssignal, das von der Erfassungsvorrichtung 86 bei im Wesentlichen vollständiger Füllung des Hohlraums 30 bzw. Füllung des Hohlraums 30 mit Flüssigkeit bis zur Marke 33 gemessen wird, kann als Referenzwert verwendet werden. Der mindestens eine erste Messwert umfasst das Intensitätssignal der Transmission der optischen Messstrahlen durch das Ausgangselement 22 bzw. entlang des Ausgangselements 22, das von der Erfassungsvorrichtung 86 erfasst wird, wenn der Füllstand der Flüssigkeit in dem Hohlraum 30 bestimmt werden soll.

Bei einer teilweisen Befüllung des Hohlraums 30 mit Flüssigkeit bzw. wenn die Flüssigkeit den in Fig. 9 horizontal dargestellten Bereich des Ausgangselements 22 nur teilweise kontaktiert, wird ein Teil der Intensität der optischen Messstrahlen ausgekoppelt bzw. gelangt aus dem Ausgangselement 22 heraus. Dennoch kann bei entsprechender Intensität der optischen Messstrahlen ein Intensitätssignal ungleich Null bzw. höher als bei im Wesentlicher vollständiger Füllung des Hohlraums 30 mit Flüssigkeit gemessen werden.

Eine Veränderung bzw. ein Ansteigen des Intensitätssignal der Transmission der optischen Messstrahlen durch das Ausgangselement 22 bzw. entlang des Ausgangselements 22 in der Erfassungsvorrichtung 86 kann durch die Vergleichsvorrichtung entsprechend erkannt werden und ein Warnsignal ausgegeben und/oder der Laserstrahl gestoppt werden. Insbesondere kann ein Warnsignal bereits dann erzeugt und/oder der Laserstrahl gestoppt werden, wenn ein Intensitätssignal ungleich Null in der Erfassungsvorrichtung 86 detektiert wird. Ein Ansteigen der Intensität bzw. des Intensitätssignals weist darauf hin, dass ein Teil des Ausgangselements 22 auf der dem Hohlraum 30 zugewandten Seite nicht mehr mit Flüssigkeit bedeckt ist.

Vorstellbar ist auch, dass optische Messstrahlen mit einer derart hohen Intensität von der Strahlerzeugungsvorrichtung 85 in das Ausgangselement 22 abgestrahlt werden, dass auch bei vollständiger Füllung des Hohlraums 30 mit Flüssigkeit ein geringes Intensitätssignal an der Erfassungsvorrichtung 86 ankommt. Somit kann mit noch höherer Zuverlässigkeit sichergestellt werden, dass auch dann, wenn nur ein kleiner Teil des (horizontal verlaufenden Teils des) Ausgangselements 22 nicht mit Flüssigkeit benetzt ist (d.h. der Hohlraum 30 ist nicht vollständig mit Flüssigkeit gefüllt oder es haben sich Luftblasen gebildet), eine Änderung des Intensitätssignals der Transmission erkannt werden kann.

Das Ausgangselement 22 kann (gegenüber der in Fig. 2a gezeigten Form) insbesondere abgerundete Kanten aufweisen, um ein Austreten von optischen Messstrahlen bei Nicht-Vorhandensein von Flüssigkeit an den Übergängen von einem in Fig. 9 horizontal verlaufenden Teil des Ausgangselements 22 in einen im Wesentlichen vertikal verlaufenden Teil des Ausgangselements 22 zu verhindern.

Der Winkel, mit dem die optischen Messstrahlen auf die Innenoberfläche des Ausgangselements 22 gelangen, bzw. der Einfallswinkel $\alpha$ liegt unterhalb eines kritischen Winkels für die interne Totalreflexion. Es gilt insbesondere $\alpha < \arcsin(n_2/n_1)$, wobei $n_1$ der Brechungsindex des Ausgangselements 22 ist und $n_2$ der Brechungsindex von Luft ist.

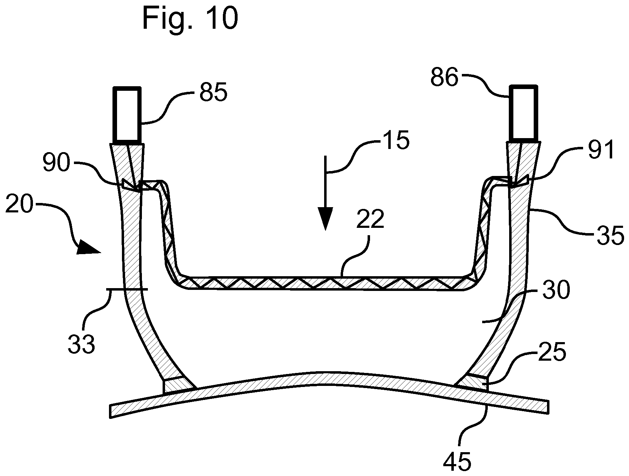

Fig. 10 zeigt eine detaillierte schematische Querschnittsansicht einer vierten Ausführungsform des Flüssigkeits-Patienteninterfaces 20 sowie eines Teils des Kurzpuls-Laser-Augenchirurgiegeräts 10, wobei der Hohlraum mit Luft gefüllt ist.

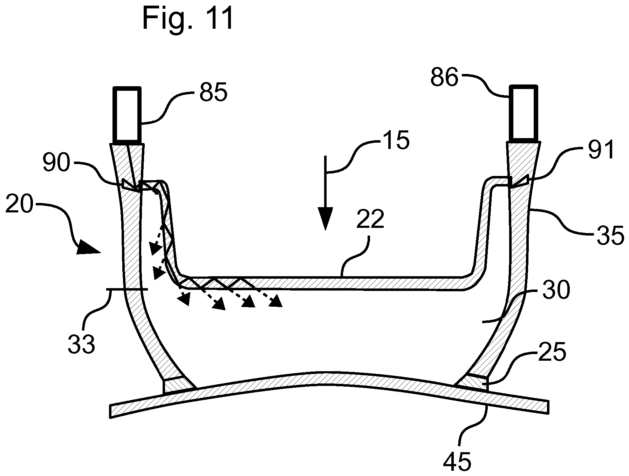

Fig. 11 zeigt eine detaillierte schematische Querschnittsansicht der vierten Ausführungsform des Flüssigkeits-Patienteninterfaces sowie eines Teils des Kurzpuls-Laser-Augenchirurgiegeräts, wobei der Hohlraum vollständig mit Flüssigkeit gefüllt ist.

In Fig. 11, bei der der Hohlraum 30 vollständig mit Flüssigkeit gefüllt ist und keine Luft mehr in dem Hohlraum 30 vorhanden ist, ist deutlich zu sehen, dass die optischen Messstrahlen aus dem Ausgangselement 22 austreten und in die Flüssigkeit gelangen (gestrichelte Linien mit Pfeilen), bis keine Intensität der optischen Messstrahlen mehr vorhanden ist. Somit wird eine Intensität bzw. ein Intensitätssignal von Null von der Erfassungsvorrichtung 86 gemessen.

Das Ausgangselement 22 in Fig. 10 bzw. Fig. 11 weist die gleiche Form wie in Fig. 9 auf. Im Gegensatz zu der in Fig. 9 gezeigten Ausführungsform ist hier die Strahlerzeugungsvorrichtung 85 zum Aussenden der optischen Messstrahlen nicht direkt bzw. unmittelbar auf dem Ausgangselement 22 angeordnet, sondern auf einem Teil der seitlichen Wand 35 des Flüssigkeits-Patienteninterfaces 20. Die optischen Messstrahlen werden durch einen Teil der seitlichen Wand 35 gestrahlt, bevor sie auf ein erstes Ablenkelement 90 gelangen. Das erste Ablenkelement 90 kann ein Prisma oder ein spiegelndes Element umfassen bzw. sein. Von dem ersten Ablenkelement 90 werden sie in einem vorgegebenen Einfallswinkel α in das Ausgangselement 22 reflektiert. Der Einfallswinkel α liegt unterhalb eines kritischen Winkels für die interne Totalreflexion. Es gilt insbesondere $\alpha < \arcsin(n_2/n_1)$, wobei $n_1$ der Brechungsindex des Ausgangselements 22 ist und $n_2$ der Brechungsindex von Luft ist. Das erste Ablenkelement 90 und das zweite Ablenkelement 91 können jeweils Bereiche bzw. Teile eines einstückig ausgebildeten Ablenkelements sein.

Nachdem die optischen Messstrahlen durch das Ausgangselement 22 gelangt sind bzw. transmittiert sind, werden die optischen Messstrahlen (wenn sie aufgrund des Vorhandenseins von Flüssigkeit an dem Ausgangselement 22 nicht bereits aus dem Ausgangselement 22 ausgetreten sind) von dem zweiten Ablenkelement 91 in Richtung des Kurzpuls-Laser-Augenchirurgiegeräts 10 reflektiert bzw. abgelenkt. Die Erfassungsvorrichtung 86, die Teil des Kurzpuls-Laser-Augenchirurgiegeräts 10 ist, erfasst die Intensität der Transmission der optischen Messstrahlen, die das Ausgangselement 22 durchlaufen haben. Eine Veränderung bzw. ein Ansteigen des Intensitätssignal kann durch die Vergleichsvorrichtung erkannt werden (d.h. weniger optische Messstrahlen verlassen das Ausgangselement 22, da sich an manchen Stellen des Ausgangselements 22 Luft und keine Flüssigkeit befindet) und der Laserstrahl gestoppt werden.

Diese Ausführungsform des Flüssigkeits-Patienteninterfaces 20 mit Ablenkelementen 90, 91 ist besonders robust ausgebildet. Zudem lässt sich das Flüssigkeits-Patienteninterface 20 technisch besonders einfach an dem Kurzpuls-Laser-Augenchirurgiegerät 10 befestigen bzw. installieren.

Die Strahlerzeugungsvorrichtung 85 kann optische Messstrahlen mit einer derartigen Wellenlänge aussenden, die von dem Ausgangselement 22 im Wesentlichen nicht absorbiert wird. Die Erfassungsvorrichtung 86 kann z.B. eine Photodiode sein. Die Erfassungsvorrichtung 86 kann auf die von der Strahlerzeugungsvorrichtung 85 zum Aussenden der optischen Messstrahlen emittierte Wellenlänge angepasst sein. Ebenso kann die Erfassungsvorrichtung 86 auf das zu erwartende Intensitätssignal bzw. Intensitätsspektrum angepasst sein.

Die Erfassungsvorrichtung 86 und die Strahlerzeugungsvorrichtung 85 zum Aussenden der optischen Messstrahlen sind nicht Teil des Flüssigkeit-Patienteninterfaces, sondern Teil des Kurzpuls-Laser-Augenchirurgiegeräts 10.

Bevorzugt erfasst die Erfassungsvorrichtung 86 nur einen kleinen Teil des Lichtspektrums, der auf die von der Strahlerzeugungsvorrichtung 85 zum Aussenden der optischen Messstrahlen ausgesandten optischen Messstrahlen angepasst ist. Hierdurch wird die von der Erfassungsvorrichtung 86 erfasste Intensität externen Lichts, z.B. Licht von der Beleuchtung des Raums, in dem die Behandlung durchgeführt wird, das in das Ausgangselement 22 gelangt, unterdrückt. Hierfür kann die Erfassungsvorrichtung 86 mit einem Filter ausgestattet sein. Wellenlängen, die nicht innerhalb eines engen Wellenlängenbands um die von der Strahlerzeugungsvorrichtung 85 zum Aussenden der optischen Messstrahlen ausgesandten Wellenlänge liegen, werden ignoriert bzw. nicht erfasst.

Alternativ oder zusätzlich können die optischen Messstrahlen von der Strahlerzeugungsvorrichtung 85 gepulst emittiert werden. Die Erfassungsvorrichtung 86 erfasst optische Messstrahlen zeitlich hierzu synchronisiert. Auf diese Weise kann das Intensitätssignal mit Hilfe des Lock-In-Verstärker-Prinzips detektiert werden bzw. Störungen minimiert werden.

Die optischen Messstrahlen der Strahlerzeugungsvorrichtung 85 können auch zusätzlich zum Beleuchten des Behandlungsbereichs des Flüssigkeit-Patienteninterfaces verwendet werden, da die optischen Messstrahlen aus dem Ausgangselement 22 in die Flüssigkeit des Hohlraums 30 austreten und somit in Richtung des zu behandelnden Auges 40 an der unteren Öffnung des Hohlraums 30 gelangen.

Die optischen Messstrahlen, die durch das Ausgangselement 22 gesandt werden, können sichtbares und/oder infrarotes Licht umfassen.

Die optischen Messstrahlen, die in das Ausgangselement 22 hinein abgestrahlt werden, können einen einzelnen optischen Messstrahl oder ein Bündel von optischen Messstrahlen aufweisen. Je nach Form des Bündels kann die Erfassungsvorrichtung 86 linienförmig (1D) oder flächig (2D) ausgebildet sein, um alle optischen Messstrahlen, die durch das Ausgangselement 22 gelangen bzw. transmittieren, zu erfassen.

Insbesondere ist vorstellbar, dass an einer Stelle des im Querschnitt senkrecht zu der Richtung des Laserstrahls 15 kreisförmig ausgebildeten Flüssigkeits-Patienteninterfaces 20 die Strahlerzeugungsvorrichtung 85 zum Aussenden der optischen Messstrahlen angeordnet ist. Über den restlichen kreisförmigen Bereich (senkrecht zu der Richtung des Laserstrahls 15) ist die Erfassungsvorrichtung 86 ausgebildet. Somit werden alle optischen Messstrahlen, die von der Strahlerzeugungsvorrichtung 85 durch das Ausgangselement 22 gelangen, erfasst, unabhängig davon, an welchem Punkt des Ausgangselements 22 entlang des Kreises sie ankommen.

Die Strahlerzeugungsvorrichtung 85 und die Erfassungsvorrichtung 86 können Teil der mechanischen Schnittstelle des Kurzpuls-Laser-Augenchirurgiegeräts 10 zu dem Flüssigkeits-Patienteninterface 20 sein bzw. in der mechanischen Schnittstelle integriert sein.

Da die Gravitation bzw. die Schwerkraft in der Regel parallel zu der Richtung des Laserstrahls 15 und nur im Ausnahmefall in einem Winkel von maximal 40° oder 45° zu der Richtung des Laserstrahls 15 verläuft, ist es, um den Füllstand der Flüssigkeit in dem Hohlraum 30 zu bestimmen, ausreichend festzustellen, ob das Ausgangselement 22 auf der dem Hohlraum 30 zugewandten Seite mit Flüssigkeit benetzt bzw. bedeckt ist. Wenn eine Stelle des Ausgangselements 22 mit Flüssigkeit bedeckt ist, ist stets in dem Bereich in Richtung des Laserstrahls 15 unterhalb der Stelle des Ausgangselements 22 Flüssigkeit bis zu der Cornea 45 vorhanden.

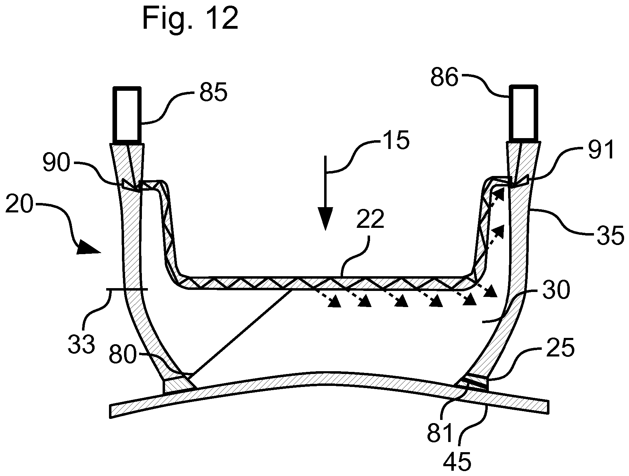

Fig. 12 zeigt eine detaillierte schematische Querschnittsansicht der vierten Ausführungsform des Flüssigkeits-Patienteninterfaces 20 sowie eines Teils des Kurzpuls-Laser-Augenchirurgiegeräts 10, wobei der Hohlraum 30 teilweise mit Flüssigkeit gefüllt ist. Die Gravitation verläuft genau oder nahezu parallel zu der Richtung des Laserstrahls 15. Allerdings besteht an einer Stelle im Bereich des Kontaktelements 25 ein Leck 81 - wie in der Figur 12 auf der rechten Seite schematisch angedeutet. Die Flüssigkeitsgrenze 80 ist in Fig. 12 zu sehen. Da zusätzlich zur Gravitation auch Grenzflächenspannungen zwischen der Flüssigkeit im Hohlraum 30 und dem Flüssigkeits-Patienteninterface 20 auftreten, wird die Flüssigkeitsgrenze 80 in der Regel, wie dargestellt, schräg verlaufen. Rechts von der Flüssigkeitsgrenze in Fig. 12 ist Flüssigkeit und links von der Flüssigkeitsgrenze ist Luft in dem Hohlraum 30. Deutlich zu sehen ist, dass die optischen Messstrahlen an den Stellen, an denen Flüssigkeit das Ausgangselement 22 benetzt bzw. bedeckt, teilweise aus dem Ausgangselement 22 austreten und in die Flüssigkeit gelangen (gestrichelte Linien mit Pfeilen). Die Intensität der optischen Messstrahlen wird bei jedem Auftreffen der optischen Messstrahlen auf Bereiche des Ausgangselements 22, an denen Flüssigkeit unterhalb des Ausgangselements 22 vorhanden ist, geringer, da ein Teil der optischen Messstrahlen das Ausgangselement 22 in Richtung des Hohlraums 30 verlässt. Somit wird eine geringe Intensität der optischen Messstrahlen in der Erfassungsvorrichtung 86 erfasst. Die Intensität bzw. das Intensitätssignal liegt zwischen einem Maximalwert, bei dem keinerlei Flüssigkeit in dem Hohlraum 30 ist, und einem Minimalwert, bei dem der Hohlraum 30 im Wesentlichen vollständig mit Flüssigkeit gefüllt.

Das Ausgangselement 22 oder das Ausgangselement 22 und ein Teil der seitlichen Wand 35 des Flüssigkeits-Patienteninterfaces 20 können als Wellenleiter verwendet werden. Bei der Bestimmung des Referenzwerts kann der Hohlraum 30 nicht nur bis zu der Marke 33 mit Flüssigkeit gefüllt sein, sondern derart vollständig mit Flüssigkeit gefüllt sein, dass überhaupt keine Luft mehr im Hohlraum 30 vorhanden ist.

Bezugszeichenliste

| | |
|---|---|
| 5 | Kurzpuls-Laser-Augenchirurgiesystem |
| 10 | Kurzpuls-Laser-Augenchirurgiegerät |
| 15 | Richtung des Laserstrahls |
| 20 | Flüssigkeits-Patienteninterface |
| 22 | Ausgangselement |
| 25 | Kontaktelement |
| 26 | Reflexionselement |
| 30 | Hohlraum |
| 33 | Marke |
| 35 | seitliche Wand des Flüssigkeits-Patienteninterfaces |
| 40 | Auge |
| 41 | Linse |
| 42 | Randbereich des Auges |
| 45 | Cornea |
| 50-57 | Beleuchtungsreflexe |
| 70 | optische Weglänge zwischen dem Ausgangselement und der Cornea entlang der optischen Achse |
| 71 | optische Weglänge zwischen dem Ausgangselement und dem Kontaktelement |
| 72, 72' | optische Weglänge zwischen dem Ausgangselement und der Cornea an einer beliebigen Stelle |
| 80 | Flüssigkeitsgrenze |
| 81 | Leck |
| 85 | Strahlerzeugungsvorrichtung |
| 86 | Erfassungsvorrichtung |
| 90 | erstes Ablenkelement |
| 91 | zweites Ablenkelement |

**Patentansprüche**

1. Kurzpuls-Laser-Augenchirurgiesystem (5) zum Behandeln eines Auges (40), umfassend

   - ein Kurzpuls-Laser-Augenchirurgiegerät (10) zum Erzeugen eines Laserstrahls zur Behandlung des Auges (40),
   - ein Flüssigkeits-Patienteninterface (20) zum Fixieren des Auges (40), wobei das Flüssigkeits-Patienteninterface (20) einen Hohlraum (30) zum Aufnehmen einer Flüssigkeit umfasst, wobei der Hohlraum (30) entlang einer Richtung (15) des Laserstrahls nach einem Ausgangselement (22) des Flüssigkeits-Patienteninterfaces (20) angeordnet ist und auf einer von dem Ausgangselement (22) abgewandten Seite offen und von dem Auge (40) verschließbar ist,

   wobei das Kurzpuls-Laser-Augenchirurgiegerät (10) folgende Merkmale umfasst:

   -- eine Strahlerzeugungsvorrichtung (85) zum Aussenden von optischen Messstrahlen aus dem Kurzpuls-Laser-Augenchirurgiegerät (10) in das Flüssigkeits-Patienteninterface (20),
   -- eine Erfassungsvorrichtung (86) zum Erfassen von Reflexion und/oder Rückstreuung und/oder Transmission der optischen Messstrahlen aus dem Flüssigkeits-Patienteninterface (20) in dem Kurzpuls-Laser-Augenchirurgiegerät (10),
   -- eine Bestimmungsvorrichtung zum Bestimmen mindestens eines ersten Messwerts aus der erfassten Reflexion und/oder Rückstreuung und/oder Transmission der optischen Messstrahlen, **gekennzeichnet dadurch, dass** das Kurzpuls-Laser-Augenchirurgiegerät weiterhin -- eine Vergleichsvorrichtung zum Vergleichen des mindestens einen ersten Messwerts mit einem Referenzwert zum Bestimmen des Füllstands der Flüssigkeit in dem Hohlraum (30)

umfasst,

wobei der Referenzwert ein fest vorgegebener Referenzwert ist oder der Referenzwert ein zweiter Messwert ist, der bei im Wesentlichen vollständiger Füllung des durch das Auge (40) verschlossenen Hohlraums (30) mit der Flüssigkeit aus Reflexion und/oder Rückstreuung und/oder Transmission von optischen Messstrahlen bestimmt wurde.

2. Kurzpuls-Laser-Augenchirurgiesystem (5) nach Anspruch 1, wobei
die Erfassungsvorrichtung (86) eine visuelle Kamera und/oder eine Infrarotkamera zum Detektieren von Reflexion und/oder Rückstreuung der optischen Messstrahlen umfasst.

3. Kurzpuls-Laser-Augenchirurgiesystem (5) nach Anspruch 2, wobei
die Bestimmungsvorrichtung eine Bildanalysevorrichtung zum Analysieren eines von der visuellen Kamera und/oder Infrarotkamera aufgenommen Bilds zum Erzeugen des mindestens einen ersten Messwerts aufweist.

4. Kurzpuls-Laser-Augenchirurgiesystem (5) nach einem der vorhergehenden Ansprüche, wobei
der mindestens eine Messwert ein zu einem zweiten Zeitpunkt aufgenommenes Bild von Intensitätswerten, insbesondere Helligkeitswerte und/oder Grauwerte, umfasst und der Referenzwert ein zu einem ersten Zeitpunkt aufgenommenen Bild von Intensitätswerten, insbesondere Helligkeitswerte und/oder Grauwerte, umfasst, wobei die Vergleichsvorrichtung zum Vergleichen des mindestens einen ersten Messwerts mit dem Referenzwert das zu dem ersten Zeitpunkt aufgenommene Bild von dem zu dem zweiten Zeitpunkt aufgenommenen Bild subtrahiert, wobei die Intensitätswerte jeweils einer Intensität der Rückstreuung und/oder Reflexion der ausgesandten optischen Messstrahlen entsprechen.

5. Kurzpuls-Laser-Augenchirurgiesystem (5) nach einem der vorhergehenden Ansprüche, wobei
der mindestens eine erste Messwert eine optische Weglänge (70, 72) zwischen dem Ausgangselement (22) und dem Auge (40) umfasst und der Referenzwert eine optische Weglänge (70, 72) zwischen dem Ausgangselement (22) und dem Auge (40) bei im Wesentlichen vollständiger Füllung des durch das Auge (40) verschlossenen Hohlraums (30) mit der Flüssigkeit ist.

6. Kurzpuls-Laser-Augenchirurgiesystem (5) nach einem der Ansprüche 1-5, wobei
der mindestens eine erste Messwert eine optische Weglänge (71) zwischen dem Ausgangselement (22) und einem Kontaktelement (25) des Flüssigkeits-Patienteninterfaces (20) zum unmittelbaren Kontaktieren des Auges (40) umfasst und der Referenzwert eine optische Weglänge (71) zwischen dem Ausgangselement (22) und dem Kontaktelement (25) des Flüssigkeits-Patienteninterfaces (20) zum unmittelbaren Kontaktieren des Auges (40) bei im Wesentlichen vollständiger Füllung des Hohlraums (30) mit der Flüssigkeit ist.

7. Kurzpuls-Laser-Augenchirurgiesystem (5) nach einem der Ansprüche 1-5, wobei

der mindestens eine erste Messwert ein Intensitätssignal umfasst, wobei das Intensitätssignal eine Intensität der Transmission der ausgesandten optischen Messstrahlen durch das Ausgangselement (22) angibt, und wobei der Referenzwert ein Intensitätssignal der Transmission der ausgesandten optischen Messstrahlen durch das Ausgangselement (22) bei im Wesentlichen vollständiger Füllung des Hohlraums (30) mit der Flüssigkeit ist.

8. Kurzpuls-Laser-Augenchirurgiesystem (5) nach einem der vorhergehenden Ansprüche, wobei
der mindestens eine erste Messwert ein Intensitätssignal umfasst, wobei das Intensitätssignal eine Intensität der Rückstreuung und/oder Reflexion der ausgesandten optischen Messstrahlen angibt.

9. Verfahren zum Bestimmen eines Füllstands einer Flüssigkeit in einem Hohlraum (30) eines Kurzpuls-Laser-Augenchirurgiesystems (5), wobei das Kurzpuls-Laser-Augenchirurgiesystem (5) ein Kurzpuls-Laser-Augenchirurgiegerät (10) zum Erzeugen eines Laserstrahls zur Behandlung eines Auges (40) und ein mit dem Kurzpuls-Laser-Augenchirurgiegerät (10) verbundenes Flüssigkeits-Patienteninterface (20) zum Fixieren des Auges (40) umfasst, wobei der Hohlraum (30) in dem Flüssigkeits-Patienteninterface (20) ausgebildet ist, wobei das Flüssigkeits-Patienteninterface (20) derart mit dem Kurzpuls-Laser-Augenchirurgiegerät (10) verbunden ist, dass der Laserstrahl durch ein Ausgangselement (22) des Flüssigkeits-Patienteninterfaces (20) in den Hohlraum (30) eintritt, wobei der auf einer dem Ausgangselement (22) abgewandten Seite offene Hohlraum (30) von dem Auge (40) verschließbar ist,

wobei das Verfahren folgende Schritte umfasst:

Aussenden von optischen Messstrahlen aus dem Kurzpuls-Laser-Augenchirurgiegerät (10) in das Flüssigkeits-Patienteninterface (20); Erfassen von Reflexion und/oder Rückstreuung und/oder Transmission der optischen Messstrahlen aus dem Flüssigkeits-Patienteninterface (20) in dem Kurzpuls-Laser-Augenchirurgiegerät (10); Bestimmen mindestens eines ersten Messwerts aus der erfassten Reflexion und/oder Rückstreuung und/oder Transmission der optischen Messstrahlen; **dadurch gekennzeichnet, dass** das Verfahren weiterhin folgenden Schritt umfasst: Vergleichen des mindestens einen ersten Messwerts mit einem Referenzwert zum Bestimmen des Füllstands der Flüssigkeit in dem Hohlraum (30), wobei der Referenzwert ein fest vorgegebener Referenzwert ist oder der Referenzwert ein zweiter Messwert ist, der bei im Wesentlichen vollständiger Füllung des durch das Auge (40) verschlossenen Hohlraums (30) mit der Flüssigkeit aus Reflexion und/oder Rückstreuung und/oder Transmission von optischen Messstrahlen bestimmt wurde.

10. Verfahren nach Anspruch 9, wobei bei dem Bestimmen des mindestens einen Messwerts eine optische Bildaufnahme des Hohlraums (30), insbesondere eine Bildaufnahme umfassend Helligkeitswerte und/oder Grauwerte, analysiert wird.

11. Verfahren nach Anspruch 9 oder 10, wobei der Referenzwert eine optische Weglänge (70, 72) zwischen dem Ausgangselement (22) des Flüssigkeits-Patienteninterfaces (20) und dem den Hohlraum (30) verschließenden Auge (40) zu einem ersten Zeitpunkt ist, an dem der Hohlraum (30) vollständig mit der Flüssigkeit gefüllt ist, und der mindestens eine erste Messwert eine optische Weglänge (70, 72) zwischen dem Ausgangselement (22) des Flüssigkeits-Patienteninterfaces (20) und dem den Hohlraum (30) verschließenden Auge (40) zu einem zweiten Zeitpunkt, an dem der Füllstand der Flüssigkeit in dem Hohlraum (30) bestimmt wird, umfasst.

12. Verfahren nach einem der Ansprüche 9-11, wobei der mindestens eine erste Messwert eine optische Weglänge (71) zwischen dem Ausgangselement (22) des Flüssigkeits-Patienteninterfaces (20) und einem Kontaktelement (25) des Flüssigkeits-Patienteninterfaces (20) zum unmittelbaren Kontaktieren des Auges (40) umfasst.

13. Verfahren nach einem der Ansprüche 9-11, wobei das Ausgangselement (22) eine Ausgangslinse umfasst und die optische Weglänge (70, 71) entlang der optischen Achse der Ausgangslinse gemessen wird.

14. Verfahren nach einem der Ansprüche 9-13, wobei

der mindestens eine erste Messwert ein Intensitätssignal umfasst, wobei das Intensitätssignal eine Intensität der Transmission der ausgesandten optischen Messstrahlen durch das Ausgangselement (22) angibt, und wobei der Referenzwert ein Intensitätssignal der Transmission der ausgesandten optischen Messstrahlen durch das Ausgangselement (22) bei im Wesentlichen vollständiger Füllung des Hohlraums (30) mit der Flüssigkeit ist.

15. Verfahren nach einem der Ansprüche 9-14, wobei

der mindestens eine erste Messwert ein Intensitätssignal umfasst, wobei das Intensitätssignal eine Intensität der Rückstreuung und/oder Reflexion der ausgesandten optischen Messstrahlen angibt.

**Claims**

1. Short-pulse laser eye surgery system (5) for treating an eye (40), comprising

- a short-pulse laser eye surgery apparatus (10) for generating a laser beam for treating the eye (40),
- a liquid/patient interface (20) for fixing the eye (40), the liquid/patient interface (20) comprising a cavity (30) for receiving a liquid, the cavity (30) being arranged downstream from an output element (22) of the liquid/patient interface (20) in a direction (15) of the laser beam and being open and sealable by the eye (40) on a side remote from the output element (22),

the short-pulse laser eye surgery apparatus (10) comprising the following features:

-- a beam generation device (85) for emitting optical measurement beams from the short-pulse laser eye surgery apparatus (10) into the liquid/patient interface (20),
-- a detection device (86) for detecting reflection and/or backscattering and/or transmission of the optical measurement beams from the liquid/patient interface (20) in the short-pulse laser eye surgery apparatus (10),
-- a determination device for determining at least one first measurement value out of the detected reflection and/or backscattering and/or transmission of the optical measurement beams,

**characterised in that** the short-pulse laser eye surgery apparatus further comprises

-- a comparison device for comparing the at least one first measurement value with a reference value to determine the fill level of the liquid in the cavity (30),

the reference value being a fixedly predetermined reference value or the reference value being a second measurement value which was determined from reflection and/or backscattering and/or transmission of optical measurement beams while the cavity (30), sealed by the eye (40), was substantially completely filled with the liquid.

2. Short-pulse laser eye surgery system (5) according to claim 1, wherein the detection device (86) comprises a visual camera and/or an infrared camera for detecting reflection and/or backscattering of the optical measurement beams.

3. Short-pulse laser eye surgery system (5) according to claim 2, wherein the determination device has an image analysis device for analysing an image captured by the visual camera and/or infrared camera to generate the at least one first measurement value.

4. Short-pulse laser eye surgery system (5) according to any of the preceding claims, wherein the at least one measurement value comprises an image, captured at a second point in time, of intensity values, in particular brightness values and/or grey values, and the reference value comprises an image, captured at a first point in time, of intensity values, in particular brightness values and/or grey values, the comparison device subtracting the image captured at the first time from the image captured at the second time so as to compare the at least one first measurement value with the reference value, the intensity values each corresponding to an intensity of the backscattering and/or reflection of the emitted optical measurement beams.

5. Short-pulse laser eye surgery system (5) according to any of the preceding claims, wherein the at least one first measurement value includes an optical path length (70, 72) between the output element (22) and the eye (40), and the reference value is an optical path length (70, 72) between the output element (22) and the eye (40) when the cavity (30), sealed by the eye (40), is substantially filled with the liquid.

6. Short-pulse laser eye surgery system (5) according to any of claims 1-5, wherein the at least one first measurement value includes an optical path length (71) between the output element (22) and a contact element (25) of the liquid/patient interface (20) for directly contacting the eye (40), and the reference value is an optical path length (71) between the output element (22) and the contact element (25) of the liquid/patient interface (20) for directly contacting the eye (40) when the cavity (30) is substantially completely filled with the liquid.

7. Short-pulse laser eye surgery system (5) according to any of claims 1-5, wherein

the at least one first measurement value includes an intensity signal, the intensity signal specifying an intensity of the transmission of the emitted optical measurement beams through the output element (22), and wherein the reference value is an intensity signal of the transmission of the emitted optical measurement beams through the output element (22) when the cavity (30) is substantially completely filled with the liquid.

8. Short-pulse laser eye surgery system (5) according to any of the preceding claims, wherein the at least one first measurement value includes an intensity signal, the intensity signal specifying an intensity of the backscattering and/or reflection of the emitted optical measurement beams.

9. Method for determining a fill level of a liquid in cavity (30) of a short-pulse laser eye surgery system (5), the short-pulse laser eye surgery system (5) comprising a short-pulse laser eye surgery apparatus (10) for generating a laser beam for treating the eye (40) and a liquid/patient interface (20), connected to the short-pulse laser eye surgery apparatus (10), for fixing the eye (40), the cavity (30) being formed in the liquid/patient interface (20), the liquid/patient interface (20) being connected to the short-pulse laser eye surgery apparatus (10) in such a way that the laser beam enters the cavity (30) through an output element (22) of the liquid/patient interface (20), the cavity (30), which is open on a side remote from the output element (22), being sealable by the eye (40), the method comprising the following steps:

emitting optical measurement beams from the short-pulse laser eye surgery apparatus (10) into the liquid/patient interface (20); detecting reflection and/or backscattering and/or transmission of the optical measurement beams from the liquid/patient interface (20) in the short-pulse laser eye surgery apparatus (10);

determining at least one first measurement value out of the detected reflection and/or backscattering and/or transmission of the optical measurement beams;

**characterised in that** the method further comprises the following step:

comparing the at least one first measurement value with a reference value to determine the fill level of the liquid in the cavity (30), the reference value being a fixedly predetermined reference value or the reference value being a second measurement value which was determined from reflection and/or backscattering and/or transmission of optical measurement beams while the cavity (30), sealed by the eye (40), was substantially completely filled with the liquid.

10. Method according to claim 9, wherein an optical image capture of the cavity (30), in particular an image capture comprising brightness values and/or grey values, is analysed during the determination of the at least one measurement value.

11. Method according to either claim 9 or claim 10, wherein the reference value is an optical path length (70, 72) between the output element (22) of the liquid/patient interface (20) and the eye (40) sealing the cavity (30) at a first point in time, at which the cavity (30) is completely filled with the liquid, and the at least one first measurement value includes an optical path length (70, 72) between the output element (22) of the liquid/patient interface (20) and the eye (40) sealing the cavity (40) at a second point in time, at which the fill level of the liquid in the cavity (30) is being determined.

12. Method according to any of claims 9-11, wherein the at least one first measurement value comprises an optical path length (71) between the output element (22) of the liquid/patient interface (20) and a contact element (25) of the liquid/patient interface (20) for directly contacting the eye (40).

13. Method according to any of claims 9-11, wherein the output element (22) comprises an output lens, and the optical path length (70, 71) is measured along the optical axis of the output lens.

14. Method according to any of claims 9-13, wherein

the at least one first measurement value includes an intensity signal, the intensity signal specifying an intensity of the transmission of the emitted optical measurement beams through the output element (22), and wherein the reference value is an intensity signal of the transmission of the emitted optical measurement beams through the output element (22) when the cavity (30) is substantially completely filled with the liquid.

15. Method according to any of claims 9-14, wherein the at least one first measurement value includes an intensity signal, the intensity signal specifying an intensity of the backscattering and/or reflection of the emitted optical measurement beams.

## Revendications

1. Système de chirurgie ophtalmique au laser à impulsions courtes (5) pour traiter un œil (40), comprenant :

    - un appareil de chirurgie ophtalmique au laser à impulsions courtes (10) pour générer un faisceau laser afin de traiter l'œil (40),
    - une interface patient liquide (20) pour immobiliser l'œil (40), l'interface patient liquide (20) comprenant une cavité (30) destinée à recevoir un liquide, la cavité (30) étant disposée le long d'une direction (15) du faisceau laser après un élément de sortie (22) de l'interface patient liquide (20) et étant ouverte sur un côté opposé à l'élément de sortie (22) et pouvant être fermée par l'œil (40),

    l'appareil de chirurgie ophtalmique au laser à impulsions courtes (10) comprenant les caractéristiques suivantes :

    -- un dispositif de génération de faisceau (85) pour émettre des faisceaux optiques de mesure à partir de l'appareil de chirurgie ophtalmique au laser à impulsions courtes (10) dans l'interface patient liquide (20),
    -- un dispositif de détection (86) pour détecter la réflexion et/ou la rétrodiffusion et/ou la transmission des faisceaux optiques de mesure provenant de l'interface patient liquide (20) dans l'appareil de chirurgie ophtalmique laser à impulsions courtes (10),
    -- un dispositif de détermination pour déterminer au moins une première valeur de mesure à partir de la réflexion et/ou de la rétrodiffusion et/ou de la transmission détectées des faisceaux optiques de mesure,

    **caractérisé en ce que** l'appareil de chirurgie ophtalmique au laser à impulsions courtes comprend en outre :

    -- un dispositif de comparaison pour comparer ladite au moins une première valeur de mesure avec une valeur de référence pour déterminer le niveau de remplissage du liquide dans la cavité (30),

la valeur de référence étant une valeur de référence prédéfinie de manière fixe ou la valeur de référence étant une deuxième valeur de mesure qui a été déterminée à partir de la réflexion et/ou de la rétrodiffusion et/ou de la transmission de faisceaux optiques de mesure lorsque la cavité (30) fermée par l'œil (40) est sensiblement complètement remplie par le liquide.

2. Système de chirurgie ophtalmique au laser à impulsions courtes (5) selon la revendication 1, dans lequel

le dispositif de détection (86) comprend une caméra visuelle et/ou une caméra infrarouge pour détecter la réflexion et/ou la rétrodiffusion des faisceaux optiques de mesure.

3. Système de chirurgie ophtalmique au laser à impulsions courtes (5) selon la revendication 2, dans lequel

le dispositif de détermination présente un dispositif d'analyse d'image pour analyser une image prise par la caméra visuelle et/ou la caméra infrarouge afin de générer ladite au moins une première valeur de mesure.

4. Système de chirurgie ophtalmique au laser à impulsions courtes (5) selon l'une des revendications précédentes, dans lequel

ladite au moins une valeur de mesure comprend une image de valeurs d'intensité, en particulier de valeurs de luminosité et/ou de valeurs de gris, prise à un deuxième instant, et la valeur de référence comprend une image de valeurs d'intensité, en particulier de valeurs de luminosité et/ou de valeurs de gris, prise à un premier instant, le dispositif de comparaison pour comparer ladite au moins une première valeur de mesure à la valeur de référence soustrait l'image prise au premier instant de l'image prise au deuxième instant, les valeurs d'intensité correspondant chaque fois à une intensité de la rétrodiffusion et/ou de la réflexion des faisceaux optiques de mesure émis.

5. Système de chirurgie ophtalmique au laser à impulsions courtes (5) selon l'une des revendications précédentes, dans lequel

ladite au moins une première valeur de mesure comprend une longueur de trajet optique (70, 72) entre l'élément de sortie (22) et l'œil (40) et la valeur de référence est une longueur de trajet optique (70, 72) entre l'élément de sortie (22) et l'œil (40) lorsque la cavité (30) fermée par l'œil (40) est sensiblement complètement remplie par le liquide.

6. Système de chirurgie ophtalmique au laser à impulsions courtes (5) selon l'une des revendications 1 à 5, dans lequel

ladite au moins une première valeur de mesure comprend une longueur de trajet optique (71) entre l'élément de sortie (22) et un élément de contact (25) de l'interface patient liquide (20) pour un contact direct avec l'œil (40) et la valeur de référence est une longueur de trajet optique (71) entre l'élément de sortie (22) et l'élément de contact (25) de l'interface patient liquide (20) pour un contact direct avec l'œil (40) lorsque la cavité (30) est sensiblement complètement remplie par le liquide.

7. Système de chirurgie ophtalmique au laser à impulsions courtes (5) selon l'une des revendications 1 à 5, dans lequel

ladite au moins une première valeur de mesure comprend un signal d'intensité, ledit signal d'intensité indiquant une intensité de la transmission des faisceaux optiques de mesure émis à travers l'élément de sortie (22), et ladite valeur de référence étant un signal d'intensité de la transmission des faisceaux optiques de mesure émis à travers l'élément de sortie (22) lorsque la cavité (30) est sensiblement complètement remplie par le liquide.

8. Système de chirurgie ophtalmique au laser à impulsions courtes (5) selon l'une des revendications précédentes, dans lequel

ladite au moins une première valeur de mesure comprend un signal d'intensité, ledit signal d'intensité indiquant une intensité de la rétrodiffusion et/ou de la réflexion des faisceaux optiques de mesure émis.

9. Procédé pour déterminer un niveau de liquide dans une cavité (30) d'un système de chirurgie ophtalmique au laser à impulsions courtes (5), le système de chirurgie ophtalmique au laser à impulsions courtes (5) comprenant un appareil de chirurgie ophtalmique au laser à impulsions courtes (10) pour générer un faisceau laser afin de traiter un œil (40) et une interface patient liquide (20) reliée à l'appareil de chirurgie ophtalmique au laser à impulsions courtes (10) pour immobiliser l'œil (40), la cavité (30) étant formée dans l'interface patient liquide (20), l'interface patient liquide (20) étant reliée à l'appareil de chirurgie ophtalmique au laser à impulsions courtes (10) de telle sorte que le faisceau laser pénètre dans la cavité (30) à travers un élément de sortie (22) de l'interface patient liquide (20), la cavité (30) ouverte sur un côté opposé à l'élément de sortie (22) pouvant être fermée par l'œil (40),

le procédé comprenant les étapes consistant à :

émettre des faisceaux optiques de mesure à partir de l'appareil de chirurgie ophtalmique au laser à impulsions courtes (10) dans l'interface patient liquide (20);

détecter la réflexion et/ou la rétrodiffusion et/ou la transmission des faisceaux optiques de mesure provenant de l'interface patient liquide (20) dans l'appareil de chirurgie ophtalmique au laser à impulsions courtes (10) ;

déterminer au moins une première valeur de mesure à partir de la réflexion et/ou de la rétrodiffusion et/ou de la transmission détectées des faisceaux optiques de mesure ;

**caractérisé en ce que** le procédé comprend en outre l'étape consistant à :

comparer ladite au moins une première valeur de mesure avec une valeur de référence pour déterminer le niveau de remplissage du liquide dans la cavité (30), la valeur de référence étant une valeur de référence prédéterminée de manière fixe ou la valeur de référence étant une deuxième valeur de mesure qui a été déterminée à partir de la réflexion et/ou de la rétrodiffusion et/ou de la transmission de faisceaux optiques de mesure lorsque la cavité (30) fermée par l'œil (40) est sensiblement complètement remplie par le liquide.

10. Procédé selon la revendication 9, dans lequel lors de la détermination de ladite au moins une valeur de mesure, une prise d'image optique de la cavité (30), en particulier une prise d'image comprenant des valeurs de luminosité et/ou des valeurs de gris, est analysée.

11. Procédé selon la revendication 9 ou 10, dans lequel la valeur de référence est une longueur de trajet optique (70, 72) entre l'élément de sortie (22) de l'interface patient liquide (20) et l'œil (40) fermant la cavité (30) à un premier instant auquel la cavité (30) est complètement remplie par le liquide, et ladite au moins une première valeur de mesure comprend une longueur de trajet optique (70, 72) entre l'élément de sortie (22) de l'interface patient liquide (20) et l'œil (40) fermant la cavité (30) à un deuxième instant auquel le niveau de remplissage du liquide dans la cavité (30) est déterminé.

12. Procédé selon l'une des revendications 9 à 11, dans lequel ladite au moins une première valeur de mesure comprend une longueur de trajet optique (71) entre l'élément de sortie (22) de l'interface patient liquide (20) et un élément de contact (25) de l'interface patient liquide (20) destiné à entrer directement en contact avec l'œil (40).

13. Procédé selon l'une des revendications 9 à 11, dans lequel l'élément de sortie (22) comprend une lentille de sortie et la longueur du trajet optique (70, 71) est mesurée le long de l'axe optique de la lentille de sortie.

14. Procédé selon l'une des revendications 9 à 13, dans lequel ladite au moins une première valeur de mesure comprend un signal d'intensité, ledit signal d'intensité indiquant une intensité de la transmission des faisceaux optiques de mesure émis à travers l'élément de sortie (22),

et ladite valeur de référence étant un signal d'intensité de la transmission des faisceaux optiques de mesure émis à travers l'élément de sortie (22) lorsque la cavité (30) est sensiblement complètement remplie par le liquide.

15. Procédé selon l'une des revendications 9 à 14, dans lequel ladite au moins une première valeur de mesure comprend un signal d'intensité, ledit signal d'intensité indiquant une intensité de la rétrodiffusion et/ou de la réflexion des faisceaux optiques de mesure émis.

## Fig. 1

## Fig. 2a

Fig. 2b

Fig. 3

15

22

70

30

45

41

Fig. 4

15

22

70

30

45

41

Fig. 5

Fig. 6

Fig. 7

Fig. 8

Fig. 9

Fig. 10

## Fig. 11

# Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2013103014 A